(19) Europäisches Patentamt
European Patent Office
Office européen des brevets

(11) EP 2 427 489 B1

(12) **EUROPEAN PATENT SPECIFICATION**

(45) Date of publication and mention of the grant of the patent:
**22.04.2015 Bulletin 2015/17**

(21) Application number: **10719531.5**

(22) Date of filing: **07.05.2010**

(51) Int Cl.:
***C07K 16/00*** *(2006.01)* ***C12N 15/81*** *(2006.01)*
***C12R 1/865*** *(2006.01)* ***C07K 16/44*** *(2006.01)*

(86) International application number:
**PCT/EP2010/056264**

(87) International publication number:
**WO 2010/128143 (11.11.2010 Gazette 2010/45)**

(54) **METHOD OF CONTROLLING O-LINKED GLYCOSYLATION OF ANTIBODIES**

VERFAHREN ZUR STEUERUNG DER O-GLYKOSILIERUNG VON ANTIKÖRPERN

PROCÉDÉ POUR LIMITER LA GLYCOSYLATION À LIAISON O DES ANTICORPS

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **07.05.2009 EP 09159642**

(43) Date of publication of application:
**14.03.2012 Bulletin 2012/11**

(73) Proprietor: **Novozymes Biopharma DK A/S**
**2880 Bagsvaerd (DK)**

(72) Inventors:
- **EVANS, Leslie Robert**
  **Nottingham,**
  **Nottinghamshire NG2 6ES (GB)**
- **HUGHES, Miranda**
  **Nottingham,**
  **Nottinghamshire NG23 5LG (GB)**
- **HAY, Joanna**
  **Colyton,**
  **Devon EX24 6RP (GB)**
- **SLEEP, Darrell**
  **Nottingham,**
  **Nottinghamshire NG2 5DS (GB)**
- **TOOTH, David John**
  **Nottingham,**
  **Nottinghamshire NG7 1FD (GB)**
- **DODSWORTH, Neil**
  **Nottingham,**
  **Nottinghamshire NG9 6JY (GB)**
- **SAXTON, Malcolm John**
  **Nottingham,**
  **Nottinghamshire NG3 5DF (GB)**
- **WATERS, Joanne Patricia**
  **Nottingham,**
  **Nottinghamshire NG5 3GZ (GB)**
- **ATHWAL, Gurdeep Singh**
  **Nottingham,**
  **Nottinghamshire NG5 1DE (GB)**
- **CAMERON, Jason**
  **Mapperley,**
  **Nottingham NG3 6HB (GB)**

(74) Representative: **Williams, Rachel Clare**
**Novozymes Biopharma UK Limited**
**Castle Court**
**59 Castle Boulevard**
**Nottingham**
**NG7 1FD (GB)**

(56) References cited:
**EP-A1- 2 022 855** **WO-A1-2005/061718**
**WO-A1-2009/105357** **WO-A2-94/29457**

- **KURODA KOUSUKE ET AL: "Efficient antibody production upon suppression of O mannosylation in the yeast Ogataea minuta." APPLIED AND ENVIRONMENTAL MICROBIOLOGY JAN 2008 LNKD- PUBMED: 18039826, vol. 74, no. 2, January 2008 (2008-01), pages 446-453, XP007913976 ISSN: 1098-5336 cited in the application**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- MILLER ET AL: "Production, purification, and characterization of human scFv antibodies expressed in Saccharomyces cerevisiae, Pichia pastoris, and Escherichia coli" PROTEIN EXPRESSION AND PURIFICATION, ACADEMIC PRESS, SAN DIEGO, CA LNKD- DOI: 10.1016/J.PEP.2005.04.015, vol. 42, no. 2, 16 July 2005 (2005-07-16), pages 255-267, XP005807879 ISSN: 1046-5928
- LENGELER K B ET AL: "Protein-O-mannosyltransferases in virulence and development" CMLS CELLULAR AND MOLECULAR LIFE SCIENCES, BIRKHÄUSER-VERLAG, BA, vol. 65, no. 4, 3 November 2007 (2007-11-03), pages 528-544, XP019583897 ISSN: 1420-9071 cited in the application

## Description

### REFERENCE TO A SEQUENCE LISTING

This application contains a Sequence Listing in computer readable form. The computer readable form is incorporated herein by reference.

### FIELD OF THE INVENTION

The present invention relates to a method for controlling O-linked glycosylation of polypeptides produced recombinantly in fungi. The invention relates in particular to the production of antibodies having a low degree of glycosylation.

### BACKGROUND OF THE INVENTION

Recombinant proteins are now widely used in many fields including; commercial and industrial enzymes, diagnostics, analytics, downstream purification, formulation, as vaccines and as therapeutic agents.

Traditionally, and prior to the advent of recombinant DNA technology, mammalian proteins have been obtained from mammalian origins. However, it follows that proteins derived from mammalian origins inevitably risk being infected or contaminated with potentially deleterious, or adventitious viruses, prions or other harmful agents. These methods have proved to be both expensive and in many cases non reproducible. Consequently, mammalian cell culture techniques have been used as a source for mammalian proteins, but culturing mammalian cells is costly, technically difficult and the yields are often modest.

For these, as well as other reasons, such as easy scale up and rapid growth rates, microbial expression systems have the potential to be viable alternatives in the production of biopharmaceutical and biotherapeutic proteins including antibodies, antibody fragments, and antibody fusions amongst other commercially relevant proteins. The production of antibodies and antibody fragments is discussed in Joosten et al., Microbial Cell Factories (2003), 2, 1-15 and Gasser and Mattanovich, Biotechnol. Lett (2007) 29, 201-212.

WO94/29457 discloses a process for producing fusion proteins comprising ScFv fragment by a transformed mould belonging to the genus Aspergillus, containing DNA encoding the ScFv fragment under control of at least one expression and/or secretion regulating region derived from a mould. It is disclosed that the order of the variable chains can be $V_H$-linker-$V_L$ or $V_L$-linker-$V_H$, but no effects of the order is suggested.

Miller et al. in Protein Expression and Purification 42 (2005) p. 255-267 discloses a study where three different microbial host organisms: *Saccharomyces cerevisiae, Pichia pastoris* and *Escherichia coli* were used for expression of ScFv fragments. It was found that S. *cerevisiae* produced hyperglycosylated ScFv, having some heterogeneity and suboptimal binding affinity, *P. pastoris* produced inconsistent levels of different ScFvs and *E. coli* produced ScFvs in inclusion bodies. The disclosure did not contain any considerations of how the glycosylation could be affected.

However, the phenomenon of O-linked glycosylation as a post-translational modification of recombinant proteins for potential pharmaceutical manufacture and therapeutics is a concern as it has implications for, among other things, the heterogeneity, stability, immunogenic (both antigenic and immunosuppressive), structure, function, activity, secretion, therapeutic efficacy, lymphoprolific effects and aggregation of the product. The structure of O-linked sugar chains in fungal expressed proteins are known to present themselves differently than that seen with mammalian expression systems. Similarly, heterologous proteins which would not normally be O-linked glycosylated may be when expressed in fungi. Hence, for the production of pharmaceutical grade proteins, e.g. therapeutic monoclonal antibodies (mAbs), it would be desirable to reduce the amount of glycosylation of mammalian proteins produced in fungi.

It is generally known that protein glycosylation is widespread amongst both prokaryotes and eukaryotes. This modification has been linked to cell wall integrity, cellular differentiation, virulence, secretion and development. Glycosyl residues can be linked to proteins via asparagine (N-glycosylation) or via hydroxylated amino acids, primarilarly serine and threonine, but more rarely, tyrosine, hydroxyproline and hydroxylysine (O-glycosylation). Various monosaccharides can be O-linked to proteins, including galactose (Gal), glucose (Glc), N-acetylgalactosamine (GalNAc) and mannose (Man). These then could be extended further by additional sugars, attached via O-glycosidic bonds, mediated by mannosyl transferases, or other specific sugar transferases. (ref: Lussier, M. et al (1999) Biochim. Biophys. Acta 1426, 323-334).

There has therefore been considerable interest in providing methods for reducing the mannose type glycosylation of mammalian proteins, such as antibodies, produced in fungi.

In the yeast *Saccharomyces cerevisiae* O-mannosylation is initiated in the endoplasmic reticulum (ER) by the transfer of mannose from dolichy phosphate mannose (Dol-P-Man) as a sugar donor to the β-hydroxyl group of an appropriate amino acid. This reaction is catalyzed by a family of protein O-mannosyltransferases (PMTs, E.C 2.4.1.109). *S. cerevisiae* has seven PMT family members (*Sc*Pmt1-7p), which exhibit similar hydropathy profiles, suggesting multiple transmembrane domains (ref: Strahl-Bolsinger, S. and Scheinost, A. (1999) J. Biol. Chem. 274, 9068-9075). ScPmt1p

through to ScPmt6p share an overall protein identity of over 55%, with ScPmt7p being conserved to a lesser extent. This PMT family is further sub-divided into subfamilies, excluding ScPmt7p, based on phylogentic analysis, (refs:Willer, et al., (2003) Curr. Opin. Struc. Biol. 13 621-630 and Lengeler et al. (2008) Cell. Mol. Life Sci. 65, 528-544), and the degree of homology within three main sequence motifs (ref: Girrbach, V., et al (2000) J. Biol. Chem. 275 19288-19296). These are classified into the PMT1, PMT2 and PMT4 subfamilies, whose members include the transferases closely related to ScPmt1p, ScPmt2p and ScPmt4p respectively. PMT1 family members (ScPmt1p and ScPmt5p) interact as dimers/pairs with the PMT2 subfamily (ScPmt2p and ScPmt3p). The PMT4 subfamily is known to form homomeric complexes (ref: Girrbach, V and Strahl, S. (2003) J. Biol. Chem. 278, 12554-12562).

Homologs of yeast PMTs have been found in filamentous fungi. For example, in *Aspergillus nidulans* three genes coding for PMTs, designated *AnPMTA, AnPMTB* and *AnPMTC. An*PmtAp, *An*PmtBp and *An*PmtCp have been assigned to the PMT2, PMT1 and PMT4 subfamilies, respectively (ref: Goto, M. (2007), Biosci. Biotechnol. Biochem. 71 1415-1427). Similarly the three Schizosaccharomyces pombe genes *OGM1*+, *OGM2*+ and *OGM4*+ are closely related to *S. cerevisiae PMT1, PMT2, PMT4* genes (Tanaka et al., (2005) Biochem. Biophys Res. Comm. 330, 813-820). *Candida albicans* contains five *PMT* genes *CaPMT1, CaPMT2, CaPMT4, CaPMT5, CaPMT6* all with homology to their respective *Saccharomyces* archetypes (Prill et al., (2005) Mol. Micro. 55, 546-560). The homology of various fungal protein O-mannosyltransferases is disclosed and discussed by Lengeler et al. (2008) Cell. Mol. Life Sci. 65, 528-544**.**

Kuroda *et. al.* have shown that suppression of O-mannosylation can be achieved using a PMT inhibitor in *Ogataea minuta* (Kuroda, K., et al (2008) Appl. Environ. Microbiol. 74 446-453).

It is not the case that disruption of *PMT1* will always bring about a reduction in O-linked glycosylation as shown by the lack of effect of *pmt1* or *pmt2* disruption on the glycosylation of human insulin-like growth factor in Saccharomyces (Finck et al., Glycobiology (1996) 6, 313-320). It is known in the art that particular *PMT* genes are associated with the modification of particular endogenous host proteins (Gentzsch and Tanner (1997) Glycobiology 7, 481-486)

The expression of the *PMT1* and *PMT2* genes are important for host cells growth and either deletion alone can adversely affect the ability of the fungal host cells to grow, thus making it difficult to produce a sufficient quantity of host cells or recombinant protein with reduced amount of O-linked glycosylation. (WO2007061631).

WO 2009/105357 discloses disruption of PMT genes in lower eukaryotes and it is concluded that in addition to deletion of PMT genes it is necessary to reduce or eliminate the function of at least one endogeneous gene encoding a chaperone protein such as protein disulphide isomerise and expression of at least one mammalian homolog of the chaperone protein in the lower eukaryotic host cell i order to obtain a reduced level of protein O-glycosylation.

There exists therefore a need to provide methods for producing antibodies in fungi having a low degree of glycosylation.

## SUMMARY OF THE INVENTION

The invention relates to the use of a $V_HV_L$ orientation of the $V_L$ and $V_H$ domains of an scFv sequence in a polypeptide to produce a polypeptide which, when expressed in a fungal host cell, has a lower level of glycosylation compared to a polypeptide which comprises the $V_L$ and $V_H$ domains of scFv sequence in the $V_LV_H$ orientation.

The inventors have surprisingly found that by the use of the invention it is possible to obtain reduced level of glycosylation of a polypeptide comprising an antibody sequence or a fragment thereof.

The disclosure further relates to polypeptides obtainable according to the invention.

Compositions comprising polypeptides prepared according to the invention are further disclosed.

## BRIEF DESCRIPTION OF DRAWINGS

Fig. 1 the plasmid map of pDB2777 described further in example 1.

Fig. 2 the plasmid map of pAYE587 described further in example 1.

Fig. 3 the plasmid map of pDB2779 described further in example 1.

Fig. 4 the plasmid map of pDB3070 described further in example 1.

Fig. 5 the plasmid map of pDB3088 described further in example 1.

Fig. 6 the plasmid map of pDB3017 described further in example 2.

Fig. 7 the plasmid map of pDB3983 described further in example 10.

## DETAILED DESCRIPTION OF THE INVENTION

The term "antibody" or "antibody molecule" as used herein is thus intended to include whole antibodies (e.g. Immunoglobulin G (IgG), Immunoglobulin A (IgA), Immunogolbulin E (IgE), Immunoglobulin M (IgM), or Immunoglobulin D (IgD), mAbs, polyclonal antibodies, and chimeric antibodies. The Immunoglobulin (Ig) classes can be further divided into subclasses on the basis of small differences in the amino acid sequences in the constant region of the heavy chains. Igs within a subclass can have similar heavy chain constant region amino acid sequences, wherein differences are detected by serological means. For example, the IgG subclasses comprise IgGI, IgG2, IgG3, and IgG4, wherein the heavy chain is classified as being a gamma 1 heavy chain, a gamma 2 heavy chain, and so on due to the amino acid differences. The light chain can also be of the kappa or lambda type. In another example, the IgA subclasses comprise IgAI and IgA2, wherein the heavy chain is classified as being an alpha 1 heavy chain or an alpha 2 heavy chain due to the amino acid differences. Antibodies in this context are also intended to include those devoid of light chains, such as those found in camel, llama and other members of the *camelidae* family, and sometimes referred to as heavy chain antibodies (HcAb). Similarly, Immunoglobulin isotype novel (or new) antigen receptors (IgNAR's), which are naturally found in cartilaginous marine animals, for example wobbegong sharks and nurse sharks, and other members of the *Chondrichthyes* class (cartilaginous fishes).

Antibody fragments which comprise an antigen binding domain are also included.

The term "antibody fragment" as used herein is intended to include any appropriate antibody fragment that displays antigen binding function. Several such antibody fragments have been described in the art and are known as Fab, F(ab')2, Fab3, scFv, Fv, dsFv, ds-scFv, Fd, dAbs, TandAbs, flexibodies dimers, minibodies, diabodies, tribodies, tetrabodies, vH domain, vL domain, $v_H$H domain, Nanobodies, , IgNAR variable single domain (v-NAR domain), fragments thereof, and multimers thereof and bispecific antibody fragments. Antibodies can be fragmented using conventional techniques. For example, F(ab')2 fragments can be generated by treating the antibody with pepsin. The resulting F(ab')2 fragment can be treated to reduce disulfide bridges to produce Fab fragments. Papain digestion can lead to the formation of Fab fragments. Fab, Fab' and F(ab')2, scFv, Fv, dsFv, Fd, dAbs, TandAbs, ds-scFv, dimers, minibodies, diabodies, bispecific antibody fragments and other fragments can also be synthesized by recombinant techniques or can be chemically synthesized. Techniques for producing antibody fragments are well known and described in the art.

Examples of preferred antibody, antibody fragment or antibody fusion include scFv, or dAb.

The antibodies, antibody fragments or antibody fusions are according to the invention produced recombinantly in a suitable host cell. The antibodies, antibody fragments or antibody fusions may be produced recombinantly in their final form or they may be produced in a form that can be converted into the final desired antibody, antibody fragment, or antibody fusion by one or more subsequent steps. For example, an antibody fragment according to the invention may be produced recombinantly as a whole antibody in a suitable host cell, and then converted into the desired antibody fragment using conventional techniques e.g. cleavage with a protease.

Preferably the antibody, antibody fragment, or antibody fusion comprise an antibody light chain variable region (vL) and/or an antibody heavy chain variable region (vH) which generally comprise the antigen binding site. In certain embodiments, the antibody, antibody fragment, or antibody fusion comprises all or a portion of a heavy chain constant region, such as an IgGI, IgG2, IgG3, IgG4, IgAI, IgA2, IgE, IgM or IgD constant region. Preferably, the heavy chain constant region is an IgGI heavy chain constant region. Furthermore, the antibody, antibody fragment or antibody fusion can comprise all or a portion of a kappa light chain constant region or a lambda light chain constant region. Preferably, the light chain constant region is a lambda light chain constant region. All or part of such constant regions may be produced naturally or may be wholly or partially synthetic. Appropriate sequences for such constant regions are well known and documented in the art.

The term "fragment" as used herein refers to fragments of biological relevance, e.g. fragments which can contribute to or enable antigen binding, e.g. from part or all of the antigen binding site, or can contribute to the inhibition or reduction in function of the antigen or can contribute to the prevention of the antigen interacting with its natural ligands. Preferred fragments thus comprise a heavy chain variable region (vH domain) and/or a light chain variable region (vL domain) of the antibodies of the invention. Other preferred fragments comprise one or more of the heavy chain complementarity determining regions (CDRs) of the antibodies of the invention (or of the vH domains of the invention), or one or more of the light chain CDRs of the antibodies of the invention (or of the vL domains of the invention). When used in the context of a nucleic acid molecule, the term "fragment" includes a nucleic acid molecule encoding a fragment as described herein.

The term "antibody sequence" is intended to mean the polypeptide sequence of an antibody comprising both CDR and framework sequences.

The phrase "immunoglobulin single variable domain" refers to an antibody variable region (vH, $v_H$H, vL) that specifically binds an antigen or epitope independently of other v regions or domains; however, as the term is used herein,

an immunoglobulin single variable domain can be present in a format (e.g., homo- or hetero-multimer) with other variable regions or variable domains where the other regions or domains are not required for antigen binding by the single immunoglobulin variable domain (i.e., where the immunoglobulin single variable domain binds antigens independently of the additional variable domains). "Immunoglobulin single variable domain" encompasses not only an isolated antibody single variable domain polypeptide, but also larger polypeptides that comprise one or more monomers of an antibody single variable domain polypeptide sequence. A "domain antibody" or "dAb" is the same as an "immunoglobulin single variable domain" polypeptide as the term is used herein. An immunoglobulin single variable domain polypeptide, as used herein refers to a mammalian immunoglobulin single variable domain polypeptide, preferably human, but also includes rodent (for example, as disclosed in WO 00/29004), camelid $v_H$H dAbs or cartilaginous marine animal-derived immunoglobulin-like molecules, for example, as disclosed in WO/2009/026638 and WO2005/118629. Camelid dAbs are immunoglobulin single variable domain polypeptides which are derived from species including camel, llama, alpaca, dromedary, and guanaco, and comprise heavy chain antibodies naturally devoid of light chain: $v_H$H- $v_H$H molecules are about ten times smaller than IgG molecules.

The term "domain" in the present disclosure, with regard to the immunogolobulins, is a folded protein structure which retains its tertiary structure independent of the rest of the protein. Generally, domains are responsible for discrete functional properties of proteins, and in many cases may be added, removed or transferred to other proteins without loss of function of the remainder of the protein and/or of the domain. A "single antibody variable domain" is a folded polypeptide domain comprising sequences characteristic of antibody variable domains. It therefore includes complete antibody variable domains and modified variable domains, for example, in which one or more loops have been replaced by sequences which are not characteristic of antibody variable domains, or antibody variable domains which have been truncated or comprise N- or C-terminal extensions, as well as folded fragments of variable domains which retain at least in part the binding activity and specificity of the full-length domain.

The term "Immunoglobulin" refers to a family of polypeptides which retain the immunoglobulin fold characteristic of antibody molecules, which contains two beta sheets and, usually, a conserved disulphide bond. Members of the immunoglobulin superfamily are involved in many aspects of cellular and non-cellular interactions *in vivo,* including widespread roles in the immune system (for example, antibodies, T-cell receptor molecules and the like), involvement in cell adhesion (for example the ICAM molecules) and intracellular signalling (for example, receptor molecules, such as the PDGF receptor). The use according to the present invention is applicable to all immunoglobulin superfamily molecules. Natural antibodies are polypeptides produced *in vivo* by an organism and which can be secreted into the plasma in response to exposure to an allergen or antigen, which polypeptides have the ability to bind specifically to said antigen. Natural antibodies include but are not limited to Igs such as IgM, IgD, IgG, IgA and IgE.

Artificial antibodies are antibodies wherein the CDRs occur together with framework sequences with which they are not naturally connected.

Artificial antibodies comprise modifications, fragments, variants, mutants, homologs and analogs or combinations of modifications, fragments, variants, mutants, homologs and analogs of natural antibodies which retain the ability to bind specifically to an antigen. Artificial antibodies include but are not limited to fragments and variants known in the art as Fab fragments, F(ab)2 fragments and scFv fragments. All have domains with Ig, or Ig-like folds, which consist of a beta sandwich of seven or more strands in two sheets with a Greek key topology.

Fusions of antibodies are according to the invention intended to mean a polypeptide comprising one or more antibody, or antibody fragment sequences and one or more sequences not derived from antibodies, which fusion is capable of binding to an antigen. Typically the one or more sequences not derived from antibodies are derived from a plasma protein such as albumins, transferrins (US2008/0220002), lactoferrins or melanotransferrins. The sequences not derived from an antibody comprise preferably at least 10 amino acids, at least 20 amino acids, 30 amino acids, more preferred at least 50 amino acids, even more preferred at least 75 amino acids and more preferred at least 100 amino acids.

The antibody fusion may be a N-terminal fusion or a C-terminal fusion or a N- and C-terminal fusion understood as a fusion where the antibody sequence is fused N-, C-or N- and C-terminally to the non antibody sequence. The antibody sequence may also be inserted internally into the non antibody sequence such as in a loop or a structure known to be located on the surface of the molecule comprising said non antibody sequence. The fusion may further comprise linker sequences between the antibody and non antibody sequences. This concept is further outlined in WO 01/79442.

In one preferred embodiment the one or more antibody sequences of an antibody fusion is an antibody fragment, such as a Fab fragment, F(ab)2 fragment and scFv fragment. In another preferred embodiment the one or more sequences not derived from antibodies is an albumin or a fragment thereof. In a particular preferred embodiment the antibody fusions comprise a scFv sequence and human serum albumin or a fragment thereof.

In another preferred embodiment the antibody is a scFv fragment or a scFv fragment fused to a plasma protein preferably albumin or a fragment thereof. A scFv fragment consists of a vL domain and a vH domain, where vL is the variable domain of the light chain and vH is the variable domain of the heavy chain expressed as a single polypeptide chain in a suitable host cell. A scFv can in principle be prepared as a vLvH fusion or as a vHvL fusion, wherein the first the vL domain is the N-terminal domain and the vH is the C-terminal domain, and the inventors have surprisingly realized

that when expression in fungal host cells the vHvL will be less glycosylated than the corresponding vLvH fusion consisting of identical domains. Thus in a particular preferred aspect the invention relates to a method for producing scFv or scFv fusions in a fungal host cell, wherein the scFv is in the vHvL domain orientation. The fungal host cell may for this aspect be any fungal host cell or may be a fungal host cell having reduced or abolished expression of the protein corresponding to the protein derived from the S. *cerevisiae pmt1* gene, and/or reduced or abolished expression of the protein corresponding to the protein derived from the S. *cerevisiae pmt4* gene in order to provide for an even lower degree of glycosylation.

Preferred framework sequences according to the invention are sequences having at least 60% sequence, preferred at least 70% identity, more preferred at least 80% identity, more preferred at least 85% identity, more preferred at least 90% identity, even more preferred at least 95% identity most preferred at least 97% identity to any one of the framework sequences from mammalian, e.g. mouse, rat, rabbit, sheep, bovine, ovine, equine, avian, primate, human; IgG, IgA, IgM, IgD or IgE or any consecutive 25 amino acid sequence fragment thereof.

Examples of framework sequences includes the framework sequences derived from IgG, IgA, IgM, IgD or IgE antibodies derived from mammals in particular from mouse, rats, rabbits, guinea pigs, camelid, shark and primates, in particular primates such as homo sapiens, and any fragments thereof. Similarly, framework sequences derived from Ig superfamilies found in members of the *camelidae* family, e.g. llamas, and members of the *Chondrichthyes* class, e.g. sharks.

As used in the art, the term "CDR" refers to a complementarity determining region within antibody variable sequences. There are usually three CDRs in each of the variable regions of the heavy chain and the light chain, which are designated CDRI, CDR2 and CDR3 for each of the variable regions. The invention is not limited to antibodies, antibody fragments, or antibody fusions with only three CDRs in each variable region.

For purposes of the present invention, the degree of identity between two amino acid sequences is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends in Genetics 16: 276-277; http://emboss.org), preferably version 3.0.0 or later. The optional parameters used are gap open penalty of 10, gap extension penalty of 0.5, and the EBLOSUM62 (EMBOSS version of BLOSUM62) substitution matrix. The output of Needle labeled "longest identity" (obtained using the -nobrief option) is used as the percent identity and is calculated as follows:

(Identical Residues x 100)/(Length of Alignment – Total Number of Gaps in Alignment)

For purposes of the present invention, the degree of identity between two deoxyribonucleotide sequences is determined using the Needleman-Wunsch algorithm (Needleman and Wunsch, 1970, *supra*) as implemented in the Needle program of the EMBOSS package (EMBOSS: The European Molecular Biology Open Software Suite, Rice et al., 2000, supra; http://emboss.org), preferably version 3.0.0 or later. The optional parameters used are gap open penalty of 10, gap extension penalty of 0.5, and the EDNAFULL (EMBOSS version of NCBI NUC4.4) substitution matrix. The output of Needle labeled "longest identity" (obtained using the -nobrief option) is used as the percent identity and is calculated as follows:

(Identical Deoxyribonucleotides x 100)/(Length of Alignment – Total Number of Gaps in Alignment).

A nucleic acid construct in relation to antibodies is according to the invention intended to be understood as a nucleic acid sequence encoding an antibody, antibody fragment, or antibody fusion in functional relation with control sequences necessary for transcription and translation of the sequence encoding said antibody, antibody fragment, or antibody fusion. The expression "in functional relation with control sequences necessary for transcription and translation of the sequence encoding said antibody, antibody fragment, or antibody fusion" is intended to mean that the sequence encoding the antibody, antibody fragment, or antibody fusion is placed in a suitable frame, distance and orientation with respect to control sequences such as promoters, ribosome binding sites, terminators, polyadenylation sites, enhancer sequences regulator sites etc. Teachings of nucleic acid constructs for expressing a polypeptide in a host cell is abundant in the prior art and the skilled person will appreciate how to apply such teaching to the present invention.

The nucleic acid construct encoding an antibody, antibody fragment, or antibody fusion in functional relation with control sequences necessary for transcription and translation in the selected host is introduced into the selected fungal host cell. Several techniques for introducing nucleic acids into a host cell exist, and the skilled person will appreciate

that the present invention is not limited to any particular such technique but any such technique may in principle be used as long as the technique is capable of introducing the nucleic acids in the host cell. An example of such a technique is a modified lithium acetate method (Sigma yeast transformation kit, YEAST-1, protocol 2; Ito et al. (1983) J. Bacteriol., 153, 16; Elble, (1992) Biotechniques, 13, 18).

The expression "capable of directing the expression of an antibody by the host cell" is intended to mean that the sequence encoding an antibody, antibody fragment or antibody fusion is provided with the necessary promoter, terminator, ribosome binding site, enhancer, polyadenylation sequences necessary for expressing the antibody, antibody fragment, or antibody fusion in the selected host cell. It is within the skills of the average practitioner to select suitable regulatory sequences for a particular host cell.

When a nucleic acid sequence encoding the modified antibody, antibody fragment, or antibody fusion according to the invention has been provided it is inserted into a suitable expression constructs as known in the art. Such an expression construct will comprise regulatory sequences in operational relation to the sequence encoding the modified antibody, antibody fragment, or antibody fusion. Teaching concerning expression of a nucleic acid sequence in a fungal host cell is available in the prior art and the skilled person will know how to apply these teachings to the present invention.

Once the construct has been prepared it is inserted into a suitable fungal host cell.

The fungal host cell may according to the invention be any fungal cell capable of expressing the antibody, antibody fragment, or antibody fusion of the invention. The fungal cell may be a filamentous fungus or an yeast. As examples of filamentous fungi can be mentioned *Aspergillus sp.*, such as *A. nidulans, A. niger, A. Awamori,* and *A. oryzae; Trichoderma sp.,* such as *T. reeseii, T. Longibrachiatum* and *T. virdee*; *Penicillum sp.* such as *P. notatum* and *P. chrysogenum; Fusidium sp., Fusarium sp., Scizophyllum sp., Mucor sp* and *Rhizopus sp.* Preferred filamentous fungi host cells include *A. niger, A. oryzae, A nidulans* and *T reeseii*

As examples of yeast host cells can be mentioned *Saccharomyces sp.* such as *S. cerevisiae* and *S. ovarum, Zygosaccharomyces sp., Schizosaccharomyces sp.* such as *S. pombe, Klyveromyces sp.* such as *K. lactis, Candida sp.* such as *C. albicans, Pichia sp, such* as *P. pastoris.* and *Hansenula sp.* Preferred yeast host cells include *S.cerevisiae, K. lactis* and *P. pastoris.*

The host cells may be wild type strains, meaning that they have a genetic configuration as could be isolated from nature or they may be genetic modified strains. Preferably the host strains have been modified genetically. Hence, the alteration, or alterations making them more suited for production of heterologous proteins. Examples of such modifications include reducing the amount of proteases expressed by the host cell, modifications that increase the capacity of the host cell to produce heterologous proteins, such as an increase of foldases, chaperones etc. Teaching in the art concerning the production of heterologous proteins in fungal cell may also be applied to the present invention.

The host cells of the invention preferably have the function of at least one endogeneous gene encoding a chaperone protein such as protein disulphide isomerise. It is preferred that the function of at least one endogeneous gene encoding a chaperone protein such as protein disulphide isomerise is not reduced but it may even be increased e.g. by inserting further copies of the gene encoding this function. Further, the host cell of the invention does preferably not comprise any mammalian homolog of the chaperone protein.

A particular preferred fungal host cell is the yeast *S. cerevisiae.* Teachings concerning the yeast host cells disclosed in the international patent application published as WO 2009/019314 also apply to the present invention.

When a host cell comprising the nucleic acid construct capable of directing the expression of an antibody, antibody fragment, or antibody fusion in the selected host cell has been provided the host cell is grown under conditions allowing the expression of the antibody, antibody fragment, or antibody fusion. The growth conditions should be selected to provide sufficient nutrients to the host cell for growth and production of the desired antibody, antibody fragment, or antibody fusion and in case that the promoter directing transcription of the antibody, antibody fragment, or antibody fusion is inducible, i.e. the activity of the promoter depends on the presence or absence of particular compounds or physical conditions, the growth conditions should be adapted to induce expression of the antibody, antibody fragment, or antibody fusion. It is within the skills of the average practitioner to select suitable growth conditions depending on the selected host cell and the nucleic acid constructs.

The host cell comprising the nucleic acid construct is grown for a certain time until the antibody has been produced in a satisfactory amount whereafter the antibody, antibody fragment, or antibody fusion is recovered using well known purification techniques. It is within the skills of the average practitioner to select suitable purification methodologies, for a specific expressed protein.

In the present application and claims the term "reduced or abolished expression" is intended to mean that the particular gene or genes is (are) expressed in a lower amount in the host cell in question, compared with the corresponding host cell that has not been modified with respect to said gene or genes. Generally a "reduced or abolished" expression of a gene is achieved by subjecting a particular cell to a modification using known techniques as known in the art. Reduced or abolished expression of a gene or genes, can be achieved using classical mutagenesis of the host cell followed by selection of a mutant having the desired reduced or abolished expression of said gene or genes, or it can be achieved using recombinant DNA technologies as known in the art, such as deletion of the gene(s) or an essential

part of the gene(s) or the regulatory sequences responsible for the expression thereof, introduction of one or more substitutions or insertions in the gene(s) leading to the expression of (a) defect or truncated gene product(s) having lower activity than the natural gene product(s), or it can be achieved using antisense technology, where at least part of the gene(s) is transcribed in the opposite direction in the host cell leading to a reduced expression of gene(s). All these techniques are known in the art and the skilled person will appreciate how to apply these techniques to the present invention.

The expression "protein corresponding to the protein derived from the *S. cerevisiae pmt1* gene" is intended to mean a protein having similar activity as the protein encoded by the *S. cerevisiae pmt1* gene or high sequence identity to the protein encoded by the *S. cerevisiae pmt1* gene.

The *S. cerevisiae pmt1* gene encodes a dolichyl-phosphate-mannose-protein mannosyltransferase (EC 2.4.1.109), which catalyzes the following reaction:

dolichyl phosphate D-mannose + protein $\rightleftarrows$ dolichyl phosphate + O-D-mannosylprotein

and any protein having similar activity is considered to be a protein corresponding to the protein derived from the *S. cerevisiae pmt1* gene. Assays for determining the activity of dolychyl-phosphate-mannose protein mannosyltransferase is known in the art.

The relatedness between two amino acid sequences or between two nucleotide sequences is described by the parameter "identity". Any proteins having a sequence identity to the protein encoded by the *S. cerevisiae pmt1* gene higher than 60%, preferably 70%, more preferred 75%, more preferred 80%, more preferred 85%, even more preferred 90%, more preferred 95%, most preferred 97% is considered a protein corresponding to the protein derived from the S. *cerevisiae pmt1* gene.

The pmt genes of *S. cerevisiae* have following properties and identities: protein sequence lengths:

| | |
|---|---|
| P33775_PMT1 | 817 |
| P31382_PMT2 | 759 |
| P47190_PMT3 | 753 |
| P46971_PMT4 | 762 |
| P52867_PMT5 | 743 |
| P42934_PMT6 | 759 |
| Q06644_PMT7 | 662 |

Program: Needle, Matrix: BLOSUM62,Gap initiation penalty: 10.0, Gap extension penalty: 0.5, Gapless Identity Matrix.
Number of exact matches divided by the total length of the alignment excluding the gaps

| Protein | PMT1 | PMT2 | PMT3 | PMT4 | PMT5 | PMT6 | PMT7 |
|---|---|---|---|---|---|---|---|
| P33775_PMT1 | 100.00 | 35.43 | 35.98 | 35.98 | 53.43 | 36.12 | 27.82 |
| P31382_PMT2 | 35.43 | 100.00 | 66.13 | 35.44 | 33.71 | 46.68 | 27.86 |
| P47190_PMT3 | 35.98 | 66.13 | 100.00 | 32.55 | 32.95 | 45.70 | 28.27 |
| P46971_PMT4 | 35.98 | 35.44 | 32.55 | 100.00 | 32.84 | 32.88 | 23.91 |
| P52867_PMT5 | 53.43 | 33.71 | 32.95 | 32.84 | 100.00 | 31.11 | 27.53 |
| P42934_PMT6 | 36.12 | 46.68 | 45.70 | 32.88 | 31.11 | 100.00 | 26.23 |
| Q06644_PMT7 | 27.82 | 27.86 | 28.27 | 23.91 | 27.53 | 26.23 | 100.00 |

Alternatively the identity can be calculated based on nucleic acids sequences. Thus , any protein encoded by a gene having a sequence identity to the *S. cerevisiae pmt1* gene of at least 60%, preferably 70%, more preferred 75%, more preferred 80%, more preferred 85%, even more preferred 90%, more preferred 95%, most preferred 97% is considered a protein corresponding to the protein derived from the *S. cerevisiae pmt1* gene.

As examples of proteins corresponding to the protein derived from the *S. cerevisiae PMT1* gene can be mentioned the protein encoded by the *S. cerevisiae* pmt1, the protein encoded by the *A. nidulans AnPMTB* gene ;*C albicans,* CaPmt1, *Cryptococcus neoformans,* CnPmt1, *P. pastoris,* PMT1,*Schizosaccharomyces cerevisiae,* Oma1., Kluyveromyces lactis, PMT1 Additional proteins corresponding to the protein derived from the S. *cerevisiae PMT1* gene can be

found in (Lengeler K.B., et al (2008), Cell Mol. Life Sci. 65 528-544).

The protein encoded by the S. *cerevisiae pmt1* gene is a preferred example of a protein corresponding to the protein derived from the S. *cerevisiae pmt1* gene.

The expression "protein corresponding to the protein derived from the S. *cerevisiae pmt4* gene" is intended to mean a protein having similar activity as the protein encoded by the S. *cerevisiae pmt4* gene or high sequence identity to the protein encoded by the *S. cerevisiae pmt4* gene.

The S. *cerevisiae pmt4* gene encodes a dolichyl-phosphate-mannose-protein mannosyltransferase (EC 2.4.1.109), which catalyzes the following reaction:

dolichyl phosphate D-mannose + protein ⇌ dolichyl phosphate + O-D-mannosylprotein

and any protein having similar activity is considered to be a protein corresponding to the protein derived from the S. *cerevisiae pmt4* gene. Assays for determining the activity of dolychyl-phosphate-mannose protein mannosyltransferase is known in the art.

The relatedness between two amino acid sequences or between two nucleotide sequences is described by the parameter "identity". Any proteins having a sequence identity to the protein encoded by the *S. cerevisiae pmt4* gene higher than 60%, preferably 70%, more preferred 75%, more preferred 80%, more preferred 85%, even more preferred 90%, more preferred 95%, most preferred 97% is considered a protein corresponding to the protein derived from the S. *cerevisiae pmt4* gene.

Alternatively the identity can be calculated based on nucleic acids sequences. Thus , any protein encoded by a gene having a sequence identity to the *S. cerevisiae pmt4* gene of at least 60%, preferably 70%, more preferred 75%, more preferred 80%, more preferred 85%, even more preferred 90%, more preferred 95%, most preferred 97% is considered a protein corresponding to the protein derived from the *S. cerevisiae pmt4* gene.

As examples of proteins corresponding to the protein derived from the *S. cerevisiae PMT4* gene can be mentioned the protein encoded by the *S. cerevisiae* pmt4, the protein encoded by the *A. nidulans AnPMTCp* gene;C. albicans, CaPmt4, *Cryptococcus neoformans,* CnPmt4, *Schizosaccharomyces pombe,* Oma4, Drosophila melanogaster, DmRT, *Homo sapien,* HsPomt1,and *Trichoderma reesei,* TrPMTI. Additional proteins corresponding to the protein derived from the S.cerevisiae pmt1 gene can be found in (Lengeler K.B., et al (2008), Cell Mol. Life Sci. 65 528-544).

When a host cell comprising the nucleic acid construct capable of directing the expression of an antibody in the selected host cell has been provided the host cell is grown under conditions allowing the expression of the antibody. The growth conditions should be selected to provide sufficient nutrients to the host cell for growth and production of the desired antibody and in case that the promoter directing transcription of the antibody is inducible, i.e. the activity of the promoter depends on the presence or absence of particular compounds of physical conditions, the growth conditions should be adapted to induce expression of the antibody. It is within the skills of the average practitioner to select suitable growth conditions depending on the selected host cell and the nucleic acid constructs.

The host cell comprising the nucleic acid construct is grown for a certain time until the antibody has been produced in a satisfactory amount whereafter the antibody is recovered using well known purification techniques.

The polypeptides prepared according to the method of the invention have reduced glycosylation in particular reduced O-linked glycosylation. This is in contrast with the prior art where it was found in WO 2009/105357 that the disruption of PMT genes in itself is insufficient to have an effect on reducing levels of protein O-glycosylation without the function of at least one endogeneous gene encoding a chaperone protein such as protein disulphide isomerise being reduced or eliminated and at least one mammalian homolog of the chaperone protein being expressed in the lower eukaryotic host cell.

Concanavalin A (Con A) is a lectin which is isolated from *Canavalia ensiformis.* Con A belongs to a group of proteins widely used in both analytical assays, and for the enrichment of glycoproteins. This is based upon their ability to selectively bind different carbohydrates moieties. Con A is a metalloprotein which preferentially binds to $\alpha$-D-mannopyranosyl, $\alpha$ -D-glucopyranosyl, and sterically related species.

## EXAMPLES

### EXAMPLE 1: DISRUPTION OF *PMT* GENES IN S. *cerevisiae*

#### Creation of DXY1 *trp1Δ*

The host strain used to create a *trp1Δ* strain was DXY1, as disclosed in S. M. Kerry-Williams et al. (1998) Yeast 14:161-169. The disruption that was created ensured the total removal of the native *TRP1* sequence from the genome.

This *trp1 Δ* strain was then used to facilitate the disruption of *PMT1* and *PMT4.*

The synthetic DNA fragment to create a *TRP1* disruption could be chemically synthesized with the DNA sequence provided in SEQ ID No.1. This chemically synthesised DNA could be digested with appropriate restriction endonuclease enzymes and ligated into an appropriate pUC19 based vector (Yanisch-Perron, et al. (1985) Gene. 33: 103-119).

Plasmid pDB2777 (Fig. 1) was a pUC19 base vector that contained a piece of DNA identical to that described in SEQ ID No.1, which was liberated from its vector backbone with the restriction endonuclease enzyme *Eco*RI to release the *TRPI* disrupting fragment. This fragment along with the vector backbone as carrier DNA was used to transform DXY1 to tryptophan auxotrophy using a modified lithium acetate method (Sigma yeast transformation kit, YEAST-1, protocol 2; Ito et al. (1983) J. Bacteriol., 153, 16; Elble, (1992) Biotechniques, 13, 18). Yeast cells from the transformation were plated onto counter selective 0.3g/l 5-fluoroanthranilic acid bactoagar plates (Toyn, et al. (2000) Yeast. 16:553-560). A tryptophan auxotroph was selected and the confirmation of the *trp1 Δ* strain genotype was confirmed by Southern blot analysis. This auxotroph as designated DXY1 *trp1 Δ.*

### Creation of the *PMT1* disrupted DXY1 strains DYP1 and DYP1 *trp1 Δ*

The synthetic DNA fragment to create this disruption could be chemically synthesized with the DNA sequence provided in SEQ ID No.2. This chemically synthesized DNA would contain the 5' of the *PMT1* gene from the *Nco*I restriction endonuclease enzyme site at bp 130 to a natural *Hind*III restriction endonuclease enzyme site within *PMT1* gene at bp 911, and then from the natural *Hind*III site at bp 1595 to the second *Nco*I restriction endonuclease enzyme site at bp 2136. This chemically synthesized DNA could be digested with appropriate restriction endonuclease enzymes and ligated into an appropriate pBR322 based vector (Bolivar, et al. (1977) Gene, 2, 95-113).

The *TRP1* auxotrophic selective marker could be chemically synthesized with the DNA sequence provided in SEQ ID No.3. This chemically synthesized DNA would contain the *Hind*III restriction endonuclease enzyme sites at either end of the *TRP1* auxotrophic selective marker to facilitate ease of cloning.

Plasmid pAYE587 (Fig. 2) was a pBR322 based vector that contained a piece of DNA identical to that described in SEQ ID No.2. This plasmid was linearised with the restriction endonuclease enzyme *Hind*III and treated with calf intestinal phosphatase to produce a vector into which was ligated a piece of DNA identical to that described in SEQ ID No.3, containing the *TRP1* auxotrophic selective marker, that had also been digested with the restriction endonuclease enzyme *Hind*III. In plasmid pDB2779 (Fig. 3) the *TRP1* auxotrophic selective marker was orientated in the opposite direction to the *PMT1* ORF. The plasmid pDB2779 was then digested with the restriction endonuclease enzyme *Nco*I to release the 2.181 kb *pmt1::TRP1* disruption fragment.

The pDB2779 2.181kb *pmt1::TRP1* disruption fragment was used along with the pDB2779 backbone, which acted as carrier DNA, to transform the yeast strain DXY1 *Δ* to tryptophan prototrophy using a modified lithium acetate method (Sigma yeast transformation kit, YEAST-1, protocol 2; Ito et al. (1983) J. Bacteriol., 153, 16; Elble, (1992) Biotechniques, 13, 18). A tryptophan prototroph was selected and the confirmation of the *pmt1::TRP1* strain genotype was confirmed by Southern blot analysis. This prototroph was designated DYP1.

To be able to disrupt multiple *PMT* genes DYP1 was also made DYP1 *pmt1::trp1 Δ.* This was achieved by removing the *TRP1* auxotrophic selective marker from the middle of the disrupted *pmt1* gene by using the 1.322kb *Nco*I fragment containing the 5' and 3' ends of *PMT1* from plasmid pAYE587.

This 1.322kb *Nco*I fragment from pAYE587, along with the vector backbone as carrier DNA was used to transform DYP1 to tryptophan auxotrophy using a modified lithium acetate method (Sigma yeast transformation kit, YEAST-1, protocol 2; Ito et al. (1983) J. Bacteriol., 153, 16; Elble, (1992) Biotechniques, 13, 18). Yeast cells from the transformation were plated onto counter selective 0.3g/l 5-fluoroanthranilic acid bactoagar plates (Toyn, et al. (2000) Yeast. 16:553-560). A tryptophan auxotroph was selected and the confirmation of the *trp1 Δ* strain genotype was confirmed by Southern blot analysis. This auxotroph was designated DYP1 *trp1 Δ.*

### Creation of the *PMT4* disrupted DXY1 strain DYP4

The synthetic DNA fragment to create a *PMT4* disruption could be chemically synthesized with the DNA sequence provided in SEQ ID No.4. This chemically synthesized DNA would contain the 5' of the *PMT4* gene from the *Bgl*II restriction endonuclease enzyme site at bp 211 to an analogous site within the sequence comparable to *PMT1,* into which an engineered *Hind*III site was created, and then from a second region analogous to the second *Hind*III in *PMT1* a second *Hind*III site was created in the 3' region of *PMT4* to the *Xba*I restriction endonuclease enzyme site at bp 2250. This chemically synthesized DNA could be digested with appropriate restriction endonuclease enzymes and ligated into an appropriate pMCS5 based vector (Hoheisel, J.(1994) BioTechniques 17(3) 456-459).

Plasmid pDB3070 (Fig. 4) was a pMCS5 based vector that contained a piece of DNA identical to that described in SEQ ID No.4. This plasmid was linearised with the restriction endonuclease enzyme *Hind*III, and treated with calf

intestinal phosphatase to produce a vector into which was ligated a piece of DNA identical to that described in SEQ ID No.3, containing the *TRP1* auxotrophic selective marker, that had also been digested with the restriction endonuclease enzyme *Hind*III. This produced the plasmid pDB3088 (Fig. 5) where the *TRP1* auxotrophic selective marker was orientated in the opposite direction to the *PMT4* ORF. The plasmid pDB3088 was then digested with the restriction endonuclease enzymes *Bgl*II and *Xba*I to release the 2.231 kb *pmt4::TRP1* disruption fragment.

The pDB3088 2.231 kb *pmt4::TRP1* disruption fragment was used along with the pDB3088 backbone, which acted as carrier DNA, to transform the yeast strain DXY1 *trp1 Δ* to tryptophan prototrophy using a modified lithium acetate method (Sigma yeast transformation kit, YEAST-1, protocol 2; Ito et al. (1983) J. Bacteriol., 153, 16; Elble, (1992) Biotechniques, 13, 18). A tryptophan prototroph was selected and the confirmation of the *pmt4::TRP1* strain genotype was confirmed by Southern blot analysis. This prototroph was designated DYP4.

To be able to disrupt multiple *PMT genes* DYP4 was also made DYP4 *pmt4::trp1 Δ.* This was achieved by removing the *TRP1* auxotrophic selective marker from the middle of the disrupted *pmt4* gene by using the 1.372kb *Bgl*II/*Xba*I fragment containing the 5' and 3' ends of *PMT4* from plasmid pDB3070.

This 11.372kb *Bgl*II/*Xba*I fragment from pDB3070 along with the vector backbone as carrier DNA was used to transform DYP4 to tryptophan auxotrophy using a modified lithium acetate method (Sigma yeast transformation kit, YEAST-1, protocol 2; Ito et al. (1983) J. Bacteriol., 153, 16; Elble, (1992) Biotechniques, 13, 18). Yeast cells from the transformation were plated onto counter selective 0.3g/l 5-fluoroanthranilic acid bactoagar plates (Toyn, et al. (2000) Yeast. 16:553-560). A tryptophan auxotroph was selected and the confirmation of the *trp1 Δ* strain genotype was confirmed by Southern blot analysis. This auxotroph was designated DYP4 *trp1 Δ.*

The method of using regions analogous to the natural *Hind*III restriction endonuclease enzyme site in *PMT1* as utilised in the *PMT4* disruption was also used to disrupt the *PMT* genes, *PMT2, PMT3, PMT5* and *PMT6,* generating the strains DYP2, DYP3, DYP5 and DYP6.

## EXAMPLE 2: PREPARATION OF THE EXPRESSION PLASMID PDB 3017

## CONSTRUCTION OF scFv ALBUMIN FUSION EXPRESSION PLASMID

FITC (also referred to as FITC8, Lantto & Ohlin, 2002, J. Biol. Chem. 277: 45108-45114) is a scFv molecule that had been expressed in *P. pastoris* and *Escherichia coli* that specifically binds to fluorescein isothiocyanate. It had been derived from a synthetic scFv library, constructed by shuffling human CDR sequences in a vHvL framework. Codon optimised DNA sequences that encoded for this protein was designed in conjunction with GeneArt GmbH.

The plasmid pDB2540 as described in US 2006/0241027 was used for site directed mutagenesis to introduce the favourable cloning site *Bsu*36I in the 3' region of the recombinant human albumin (rHA) DNA sequence. Also an extra mutation was achieved that destroyed the extra *Hind*III site that was normally found next to the <u>TAATAA</u> stop codons. The mutagenic primers used along with the Stratagene Quickchange™ Site directed mutagenesis kit were LES21 & LES22;

LES21
5'-gttggtcgctgcttcccaagctgccttaggtttgtaataagcttaattcttatg-3' (SEQ ID NO: 5)

LES22
5'-cataagaattaagcttattacaaacctaaggcagcttgggaagcagcgaccaac-3' (SEQ ID NO: 6)

This produced the plasmid pDB2836. The plasmid pDB2836 was then digested with the restriction endonuclease enzymes *Xba*I and *Bsu*36I and the 0.467kb was liberated along with a *Bsu*36I/*Nde*I 1.437kb fragment from pDB2575 (as previously described in US 2006/0241027) which were then both ligated into the vector plasmid pDB2541 (as previously described in US 2006/0241027) that had similarly been digested with the restriction endonuclease enzymes *Xba*I and *Nde*I to produce a 3.353kb vector. This ligation produced the plasmid pDB2839. Plasmid pDB2839 was digested with the restriction endonuclease enzymes *Sph*I and *Nde*I to produce a 5.671 kb vector into which was ligated a 0.867kb insert that had been digested from pDB2540 (as described above) with the restriction endonuclease enzymes *Sph*I and *Nde*I also. This ligation produced the plasmid pDB2843. The oligo linkers Fwd rHA single FLAG® and Rev rHA single FLAG® were annealed and ligated into the 6.179kb pDB2843 vector that had been digested with the restriction endonuclease enzymes *Hind*III partial (at position 3005) and *Bsu*36I. This ligation created the plasmid pDB2975.

Fwd rHA single FLAG®
5'- TTAGGCTTAGATTATAAAGATGATGACGATAAATAATA-3' (SEQ ID NO: 7)
Rev rHA single FLAG®
5'-AGCTTATTATTTATCGTCATCATCTTTATAATCTAAGCC-3' (SEQ ID NO: 8)

Overlapping oligonucleotide primers were used to create a synthetic DNA encoding the 3' of the invertase leader sequence operationally linked to the FITC (vHvL) which was codon optimised for expression in S. *cerevisiae* which was then operationally linked to the 5' of the rHA.

SEQ ID No. 9 is a DNA sequence based on the 3' of the invertase leader sequence operationally linked to the FITC (vHvL) which is codon optimised for expression in S. *cerevisiae* which is then operationally linked to the 5' of the rHA. The sequence is flanked by *Bgl*II and *Cla*I restriction endonuclease sites to facilitate cloning. SEQ ID No. 9 comprises a *Bgl*II restriction endonuclease enzyme cloning site to the 3' invertase leader (signal) protein encoding sequence (nucleotides 1-11); the FITC (vHvL) protein encoding sequence which is codon optimised for expression in S. *cerevisiae* (nucleotides 12-743); the 5' rHA protein encoding sequence up to an *Cla*I restriction endonuclease enzyme cloning site (nucleotides 744-770).

The synthetic DNA encoding the 3' of the invertase leader sequence operationally linked to the FITC (vHvL) which was codon optimised for expression in S. *cerevisiae* which was then operationally linked to the 5' of the rHA was digested with the restriction endonuclease enzymes *Bgl*II and *Cla*I to produce a 0.796kb fragment; pDB2975 was digested with the restriction endonuclease enzymes *Cla*I and *Sph*I to produce a 1.950kb fragment; pDB2923 was digested with the restriction endonuclease enzymes *Bgl*II and *Sph*I to produce the 4.214kb vector; all three were used in a three way ligation to produce the sub-cloning plasmid pDB3006. The vector pDB2923 was created as follows; pDB2243 (as previously described in WO 00/44772) was digested with the restriction endonuclease enzymes *Bsu*36I and *Nde*I to produce a 1.088kb fragment, pDB2836 (as described previously) was digested with the restriction endonuclease enzymes *Bsu*36I and *Xba*I to produce a 0.467kb fragment which were both ligated into the vector pDB2541 (as described previously) that has been digested with the restriction endonuclease enzymes *Nde*I and *Xba*I to produce the 3.353kb vector. This three way ligation produced the plasmid pDB2840. The plasmid pDB2840 was digested with the restriction endonuclease enzymes *Sph*I and *Nde*I to produce a 5.326kb vector, into which was ligated a 0.885kb *Sph*I/*Nde*I insert from pDB2540 (as described previously). This ligation produced the plasmid pDB2844. The oligo linkers CF138 and CF139 were annealed and ligated into the 6.193kb pDB2844 vector that had been digested with the restriction endonuclease enzyme *Hind*III partial and treated with calf alkaline phosphatase. This ligation created the plasmid pDB2923 which was utilised above.

CF138

5'-AGCTTAACCTAATTCTAACAAGCAAAGATGCTTTTGCAAGCCTTCCTTTTCCTT

TTGGCTGGTTTTGCAGCCAAGATCTCTGCAGAAGACA-3' (SEQ ID NO: 10)

CF139

5'-AGCTTGTCTTCTGCAGAGATCTTGGCTGCAAAACCAGCCAAAAGGAAAAGGA

AGGCTTGCAAAAGCATCTTTGCTTGTTAGAATTAGGTTA-3' (SEQ ID NO: 11)

The sub-cloning plasmid pDB3006 was digested with the restriction endonuclease enzyme *Not*I and the 3.732kb expression cassette was ligated into pSAC35 that had been digested with the restriction endonuclease enzyme *Not*I and using calf intestinal phosphatase to produce the 16.303kb plasmid pDB3017 (Fig. 6) that had the FITC8 (vHvL)-rHA expression cassette in the same orientation to the *LEU2* gene.

**Example 3: Transformation of the FITC8 (vHvL)-rHA expression plasmid pDB3017 into the yeast strains DXY1, DYP1- DYP6**

The host strains used were DXY1 (S. M. Kerry-Williams et al. (1998) Yeast 14:161-1690), DYP1 - DYP6. DXY1, DYP1 - DYP6 were transformed to leucine prototrophy with the FITC8 (vHvL)-rHA expression plasmid pDB3017. Yeast were transformed using a modified lithium acetate method (Sigma yeast transformation kit, YEAST-1, protocol 2; Ito et al. (1983) J. Bacteriol., 153, 16; Elble, (1992) Biotechniques, 13, 18). Transformants were selected on BMMD-agar plates, and subsequently patched out on BMMD-agar plates. The composition of BMMD is described by Sleep et al., (2002), Yeast, 18, 403. Cryopreserved stocks were prepared in 20% (w/v) trehalose from 10mL BMMD shake flask cultures (24 hrs, 30°C, 200rpm).

**Example 4: Fermentation**

Fed-batch fermentations were carried out in a 10 L Sartorius Biostat C fermenter at 30°C; pH was monitored and adjusted by the addition of ammonia or sulphuric acid as appropriate. The ammonia also provided the nitrogen source for the cultures. The level of dissolved oxygen was monitored and linked to the stirrer speed, to maintain the level at >20% of saturation. Inocula were grown in shake flasks in buffered minimal media. For the batch-phase the cultures were inoculated into fermenter media (approximately 50% of the fermenter volume) containing 2% (w/v) sucrose. The feed stage was automatically triggered by a sharp rise in the level of dissolved oxygen. Sucrose was kept at growth-limiting concentrations by controlling the rate of feed to a set nominal growth rate. The feed consisted of fermentation media containing 50% (w/v) sucrose, all essentially as described by Collins. (Collins, S.H., (1990) Production of secreted proteins in yeast, in: T.J.R. Harris (Ed.) Protein production by biotechnology, Elsevier, London, pp. 61-77).

All fermentations were completed successfully and were good or perfect fermentations.

| Strain [plasmid] | Batch No | CDW | comment | Batch Phase (Hrs) | final % sn | Conductivity | Titre mg/ml |
|---|---|---|---|---|---|---|---|
| DXY1 [3017] | 20083E008-01 | 104.3 | Good fermentation feed rate capped at 2.7ml/min. | 51 | 57.5 | 8.02 | 5.08 |
| DYP1 [3017] | 20086E004-01 | 103.1 | Perfect fermentation | 56 | 57.5 | 8.08 | 5.5 |
| DYP2 [3017] | 20085E005-01 | 97.0 | Good fermentation slow in batch phase | 134 | 57.5 | 12.44 | 4.17 |
| DYP3 [3017] | 20083E007-01 | 104 | Good fermentation. | 51 | 55 | 8.98 | 4.03 |
| DYP4 [3017] | 20086E006-01 | 110.2 | Perfect fermentation | 56 | 51.25 | 7.24 | 5.32 |
| DYP5 [3017] | 20084E009-01 | 94.7 | Perfect fermentation | 54 | 60 | 9.15 | 4.3 |
| DYP6 [3017] | 20085E007-01 | 100.7 | Perfect fermentation | 51 | 57.5 | 9.36 | 4.1 |

**Example 5: Protein purification**

Gel Permeation High Pressure Liquid Chromatography (GP-HPLC)

Protein concentrations were determined by GP-HPLC using a LC2010 HPLC system (Shimadzu) equipped with UV detection under Shimadzu VP7.3 client server software control. Injections of 25μL were made onto a 7.8mm id x 300mm length TSK G3000SWXL column (Tosoh Bioscience), with a 6.0mm id x 40mm length TSK SW guard column (Tosoh Bioscience). Samples were chromatographed in 25mM sodium phosphate, 100mM sodium sulphate, 0.05% (w/v) sodium azide, pH 7.0 at 1 $mL.min^{-1}$, with a run time of 20 minutes. Samples were quantified by UV detection at 280nm, by peak area, relative to a recombinant human albumin standard of known concentration (10mg/mL) and corrected for their relative extinction coefficients.

Supernatant clarification

Culture supernatants from high cell density fed batch fermentations of the *S. cerevicea* strains DXY1, DYP1, DPY2, DYP3, DYP4, DYP5 and DYP6, expressing the anti FITC scFv (vHvL)-rHA-FLAG protein were harvested by standard centrifugation, using a Sorvall RC 3C centrifuge (DuPont).

Protein Purification, Diafiltration and Concentration Steps

A three step chromatography procedure for each strain was used to prepare material suitable for bioanalysis, as described herein.

The first step uses a column (bed volume approximately 400mL, bed height 11cm) packed with AlbuPure™

matrix (ProMetic). This was equilibrated with 50mM sodium phosphate, pH 6.0 and loaded with neat culture supernatants, at approximately pH 5.5-6.5, to approximately 20mg fusion/mL matrix. The column was washed with approximately 5 column volumes each of 50mM sodium phosphate, pH 6.0, 50mM sodium phosphate, pH 7.0 and 50mM ammonium acetate, pH 8.0, respectively. Bound protein was eluted using approximately two column volumes of 50mM ammonium acetate, 10mM octanoate, pH 7.0. The flow rate for the whole step was 154mL/min.

For the second step, the eluates from the first step were diluted approximately two fold with water to give a conductivity of 2.5±0.5 mS/cm after adjustment to pH 5.5±0.3 with acetic acid. This was loaded onto a DEAE-Sepharose Fast Flow (GE Healthcare) column (bed volume approximately 400mL, bed height 11cm), equilibrated with 80mM sodium acetate, 5mM octanoate, pH 5.5. Loading was approximately 30mg fusion/mL matrix. The column was washed with approximately 5 column volumes of 80mM sodium acetate, 5mM octanoate, pH 5.5. Followed by approximately 10 column volumes of 15.7mM potassium tetraborate, pH 9.2. The bound protein was eluted using two column volumes of 110mM potassium tetraborate, 200mM sodium chloride, approximately pH 9.0. The flow rate was 183mL/min during the load and wash steps, and 169mL/min during the elution.

The eluates were concentrated and diafiltered against 20mM Tris-HCL, 500mM sodium chloride, pH 7.4, using a Pall Centramate Omega 10,000 NMWCO membrane, to give a final protein concentration of approximately 100mg/mL.

For the third step, the concentrated and diafiltered eluates from the second step were adjusted with the addition of magnesium, calcium and manganese ions, to a final concentration of 1 mM, respectively. This was loaded onto a Con A Sepharose 4B (GE Healthcare) column (bed volume approximately 160mL, bed height 30cm) at approximately 10mg/mL matrix, equilibrated with 20mM Tris-HCL, 500mM sodium chloride, 1 mM magnesium chloride, 1 mM manganese chloride, 1 mM calcium chloride, pH 7.4. Unbound protein was eluted with 20mM Tris-HCL, 500mM sodium chloride, 1 mM magnesium chloride, 1 mM manganese chloride, 1 mM calcium chloride, pH 7.4. Bound protein was eluted with 20mM Tris-HCL, 500mM sodium chloride, 500mM methyl manno-pyranoside, 1 mM magnesium chloride, 1 mM manganese chloride, 1 mM calcium chloride, pH 7.4. The flow rate during the load and elutions was 7.2mL/min.

Samples were, when necessary, concentrated and diafiltered against 20mM Tris-HCL, 500mM sodium chloride, pH 7.4, using a Pall Centramate Omega 10,000 NMWCO membrane, to give a final protein concentration of approximately 10mg/mL.

The table below summarizes representative %recovery of both the bound and unbound anti FITC scFv (vHvL)-rHA-FLAG proteins, from the third step in the purification protocol, using the Con A Sepharose 4B matrix.

| Strain | Unbound % Recovery | Bound % Recovery |
|---|---|---|
| DXY1 | 78 | 5.2 |
| DYP1 | 87 | 3.3 |
| DYP2 | 89 | 5.1 |
| DYP3 | 79 | 5.6 |
| DYP4 | 81 | 2.7 |
| DYP5 | 76 | 5.1 |
| DYP6 | 87 | 5.7 |

**Example 6: Glycosylation of FITC-HSA in different pmt strains**

Method

Con A binds molecules which contain D-mannopyranosyl, D-glucopyranosyl and sterically related residues. Con A Sepharose (GE Healthcare, Bucks,UK) affinity chromatography was used to isolate mannosylated proteins from recombinant scFv albumin fusions:

3% (w/v) (approx.) scFv albumin fusions were diluted 1:1 with Con A dilution buffer (200mM sodium acetate, 85mM sodium chloride, 2mM magnesium chloride, 2mM manganese chloride, 2mM calcium chloride ph5.5 (All Fisher Scientific Analytical Grade, Loughborough, UK), and 350microL (~5mg) loaded onto an equilibrated 2mL Con A Sepharose column which was then washed (5 x 4mL) with Con A equilibration buffer (100mM sodium acetate, 100mM sodium chloride, 1 mM magnesium chloride, 1 mM manganese chloride, 1 mM calcium chloride ph5.5 (All Fisher Scientific Analytical Grade, Loughborough, UK). The column was eluted with 6mL Con A elution buffer (100mM sodium acetate, 100mM sodium chloride, 0.5M methyl-$\alpha$-D-mannopyranoside ph5.5 (All Fisher Scientific Analytical Grade, Loughborough, UK). Triplicate columns were run for each sample.

Con A loads and eluates were quantified by Bradford assay using a rHA standard curve and the Con A binding material recovered in the eluate expressed as a percentage of the load.
The rHA standard used was Recombumin® available from Novozymes Biopharma UK.

Results

| Strain | Overall mean recovery (%w/w) | Standard Deviation | Overall Mean recovery (% change from DXY1) |
|---|---|---|---|
| DXY1 | 7.88 | 0.10 | 0 |
| DYP1 | 5.81 | 0.18 | -26.22 |
| DYP2 | 7.78 | 0.26 | -1.18 |
| DYP3 | 9.03 | 0.41 | 14.68 |
| DYP4 | 5.64 | 0.27 | -28.34 |
| DYP5 | 8.71 | 0.02 | 10.64 |
| DYP6 | 7.91 | 0.4 | 0.39 |

When rHA was applied to the Con A matrix almost no binding was observed indicating that the observed binding was caused by the scFv part and not the albumin part of the fusion molecule.
DYP1 and DYP4 expressed anti-FITC fusion showed a clear reduction in mannosylation when compared to DXY1 expressed material. DYP2 and DYP6 expressed material appear to have no effect of Con A binding when compared to DXY1; DYP3 and DYP5 appear to increase the levels of mannosylation.
From this data DYP1 and DYP4 show a reduced percentage of expressed fusion molecules which are mannosylated.

**Example 7: DOMAIN ORIENTATION OF scFv's AFFECT GLYCOSYLATION**

In example 2, the preparation of the FITC-rHA fusion in the vHvL orientation was disclosed. The construction of the two unfused scFv in either the vHvL or the vLvH orientation was done accordingly (data not shown).
The host strain, Strain A as disclosed in WO2005/061718 was transformed to leucine prototrophy with the FITC scFv expression plasmids. Yeast was transformed using a modified lithium acetate method (Sigma yeast transformation kit, YEAST-1, protocol 2; Ito et al. (1983) J. Bacteriol., 153, 16; Elble, (1992) Biotechniques, 13, 18). Transformants were selected on BMMD-agar plates, and subsequently patched out on BMMD-agar plates. The composition of BMMD is described by Sleep et al., (2002), Yeast, 18, 403. Cryopreserved stocks were prepared in 20% (w/v) trehalose from 10mL BMMD shake flask cultures (24 hrs, 30°C, 200rpm).
Transformants were cultivated as shake flask cultures.10mL media in 50mL conical flasks were grown at 30°C, 200rpm with YEPD medium (1% (w/v) yeast extract, 2% (w/v) Bactopeptone, 2% (w/v) glucose), or in BMMD (buffered minimal medium, 0.67% (w/v) Bacto Yeast Nitrogen Base without amino acids, 36mM citric acid/126mM disodium hydrogen orthophosphate, pH 6.5, 2% (w/v) glucose).
Transformants were cultured for 5 days in 10mL BMMD shake flask. After centrifugation of the suspension the culture supernatant were assayed for secreted proteins, and quantified using a rHA standard.
Protein concentrations were determined by GP-HPLC using a LC2010 HPLC system (Shimadzu) equipped with UV detection under Shimadzu VP7.3 client server software control. Injections of 25$\mu$L were made onto a 7.8mm id x 300mm length TSK G3000SWXL column (Tosoh Bioscience), with a 6.0mm id x 40mm length TSK SW guard column (Tosoh Bioscience). Samples were chromatographed in 25mM sodium phosphate, 100mM sodium sulphate, 0.05% (w/v) sodium azide, pH 7.0 at 1 mL.min$^{-1}$, with a run time of 20 minutes. Samples were quantified by UV detection at 280nm, by peak area, relative to a recombinant human albumin standard of known concentration (10mg/mL) and corrected for their relative extinction coefficients.
Culture supernatants were purified and analysed as essential described in Example 5, with the appropriate scaling of the protocols. The vHvL scFv, vLvH scFv, and vHvL scFv-rHA were analysed for binding to a Con A matrix in order to determine the level of glycosylation.
The ConA binding assay showed reduced ConA binding of vHvL anti-FITC scFv, when compared to vLvH anti-FITC scFv. The data also showed that anti-FITC scFv had reduced ConA binding when fused with albumin, when compared to the unfused molecules.

| Molecule | % w/w bound to ConA column |
|---|---|
| scFv (vHvl)-rHA | 3.3 |
| scFv (vHvL) | 6.0 |
| scFv (vLvH) | 11.0 |

**Example 8: THE EFFECT OF CHAPERONE PROTEIN COPY NUMBER AND DOMAIN ORIENTATION OF scFv's ON GLYCOSYLATION**

The two host strains described in this example are as previously disclosed, in WO2005/061718, and example 1 herein, respectively.

The methodology to generate the data shown in this example has been previously described in example 7, for Strain A data, and in example 5 for DXY1 strain data. Essential, the scFvs in either the vHvL or vLvH orientation, and either fused or unfused with albumin were fermented in the stated host strains. The expressed scFvs were purified and assayed for ConA binding, to establish the level of glycosylation.

The ConA binding assay showed that as with example 7, which only showed data generated from the host strain, Strain A, the additional data points with both Strain A and DXY1 strains respectively, showed a similar trend, as seen in example 7. The addition of extra copies of an endogenous chaperone do not affect the trends for both the orientation of the scFv chains, and the fusion of albumin having an effect on glycosylation, as described in example 7.

| Molecule | Strain | % w/w bound to Con A column |
|---|---|---|
| scFv(vHvL)-rHA | Strain A | 3.3 |
| scFv(vLvH)-rHA | Strain A | 20.7 |
| scFv(vHvL)-rHA | DXY1 | 6.9 |
| scFv(vLvH)-rHA | DXY1 | 20 |
| scFv(vHvL) | Strain A | 6 |
| scFv(vLvH) | Strain A | 11 |
| scFv(vHvL) | DXY1 | 8.9 |
| scFv(vLvH) | DXY1 | 22.1 |

**Example 9: THE EFFECT OF PMT DISRUPTION AND DOMAIN ORIENTATION OF scFv's ON GLYCOSYLATION**

The three host strains described in this example are as previously described in example 1 herein, respectively.

The methodology to generate the data shown in this example has been previously described in examples 5 and 6, respectively. Essential, the scFvs in either the vHvL or vLvH orientation, and either fused or unfused with albumin were fermented in the stated host strains. The expressed scFvs were purified and assayed for ConA binding, to establish the level of glycosylation.

The ConA binding assay data shows that the orientation of the vH and vL chains does have an influence on the level of glycosylation. The level of glycosylation is consistently higher when the scFvs are expressed in the vLvH orientation, and the trend is independent of the host strain being PMT disrupted, or in a non-disrupted background. However, it is again clear that both the disruption of PMT1 and/or PMT4 does reduce the level of glycosylation, and this trend is also again independent of the scFv orientation.

| Molecule | Strain | % w/w bound to Con A column |
|---|---|---|
| scFv(vHvL)-rHA | DXY1 | 6.9 |
| scFv(vHvL)-rHA | DYP1 | 5.1 |
| scFv(vHvL)-rHA | DYP4 | 4.9 |

| | | |
|---|---|---|
| scFv(vLvH)-rHA | DXY1 | 20 |
| scFv(vLvH)-rHA | DYP1 | 6.4 |
| scFv(vLvH)-rHA | DYP4 | 9.3 |
| scFv(vHvL) | DXY1 | 8.9 |
| scFv(vHvL) | DYP1 | 5.8 |
| scFv(vHvL) | DYP4 | 6.2 |
| scFv(vLvH) | DXY1 | 22.1 |
| scFv(vLvH) | DYP1 | 8.1 |
| scFv(vLvH) | DYP4 | 9.6 |

**Example10: PREPARATION OF THE EXPRESSION PLASMID pDB3983**

**CONSTRUCTION OF A dAB ALBUMIN FUSION EXPRESSION PLASMID**

Construction of the antibody fragment-albumin fusion expression plasmid pDB3983 was essentially done using methodologies as previously described in detail for example 2. For this experiment a dAB, derived from an antibody of the kappa type, having the SEQ ID NO: 12 was produced.

Briefly, the invertase leader sequence-'antibody fragment of choice (in this case dAB)'-rHA-FLAG® expression cassette was made by isolating the 3'-invertase leader-antibody fragment-5'-rHA sequence from the plasmid, on a restriction endonuclease enzyme BglII/ClaI cut fragment, and ligating into the 3'-HSA-FLAG® restriction endonuclease enzyme BglII/ClaI cut vector.

Expression plasmids were made by ligating the restriction endonuclease enzyme NotI expression cassette into pSAC35 that had been restriction endonuclease enzyme NotI digested and treated with shrimp intestinal phosphatase. This ligation produced the plasmid stated in Fig. 7, which contained the antibody fragment-rHA fusion ORF in the same direction as the LEU2 gene.

The expression plasmid pDB3983 was used to transform the yeast strains DXY1, DYP1 and DYP4 to leucine prototrophy using the Sigma Yeast Transformation Kit, and described in detail in example 3. Similarly, fermentation, purification and ConA analysis methodologies are described in detail in examples 4,5 and 6, respectively.

The ConA binding assay data shows the dAB albumin fusion also shows a significant reduction in glycosylation, in the two PMT disrupted strains, DYP1 and DPY4, respectively, relative to the control host strain DXY1.

| Molecule | Strain | % w/w bound to Con A column |
|---|---|---|
| dAB-rHSA | DXY1 | 2.5 |
| dAB-rHSA | DYP1 | 1.1 |
| dAB-rHSA | DYP4 | 1.7 |

## Claims

1. Use of a $V_HV_L$ orientation of the $V_L$ and $V_H$ domains of an scFv sequence in a polypeptide to produce a polypeptide which, when expressed in a fungal host cell, has a lower level of glycosylation compared to a polypeptide which comprises the $V_L$ and $V_H$ domains of scFv sequence in the $V_LV_H$ orientation.

2. The use of claim 1, wherein the polypeptide is a scFv or a fusion of scFv to a plasma protein or a fragment thereof wherein the fragment comprises at least 100 amino acids.

3. The use of claim 2 wherein the plasma protein is albumin or a fragment thereof, wherein the fragment comprises at least 100 amino acids.

4. The use of any of claims 1 to 3, wherein the fungal host cell is selected among: *Aspergillus sp., Trichoderma sp., Penicillum sp. Fusidium sp., Fusarium sp., Scizophyllum sp., Mucor sp* and *Rhizopus sp.*

5. The use of claim 4 wherein the *Aspergillus sp. Is selected from A. nidulans, A. niger, A. Awamori,* and *A. oryzae.*

6. The use of claim 4 wherein the Trichoderma sp. Is selected from *T. reeseii, T. Longibrachiatum* and *T. virdee.*

7. The use of claim 4, wherein the *Penicillum sp.* is selected from *P. notatum* and *P. chrysogenum.*

8. The use of any of claims 1 to 3, wherein the fungal host cell is selected among: *Saccharomyces sp., Schizosaccharomyces sp., Zygosaccharomyces sp., Klyveromyces sp., Candida sp., Pichia sp.,* and *Hansenula sp.*

9. The use of claim 8 wherein the *Saccharomyces sp* is selected from S. *cerevisiae* and S. *ovarum.*

10. The use of claim 8 wherein the, *Schizosaccharomyces sp.* is S. *pombe.*

11. The use of claim 8 wherein the *Klyveromyces sp.* is *K. lactis.*

12. The use of claim 8 wherein the *Candida sp.* is *C. albicans.*

## Patentansprüche

1. Verwendung einer $V_HV_L$-Orientierung der $V_L$- und $V_H$-Domänen einer scFv-Sequenz in einem Polypeptid, um ein Polypeptid herzustellen, das, wenn in einer Pilzwirtszelle exprimiert, einen geringeren Glykosylierungsgrad aufweist verglichen zu einem Polypeptid, das die $V_L$- und $V_H$-Domänen der scFv-Sequenz in der $V_LV_H$-Orientierung hat.

2. Verwendung nach Anspruch 1, wobei das Polypeptid ein scFv oder eine Fusion von scFv mit einem Plasmaprotein oder einem Fragment davon ist, wobei das Fragment mindestens 100 Aminosäuren umfasst.

3. Verwendung nach Anspruch 2, wobei das Plasmaprotein Albumin oder ein Fragment davon ist, wobei das Fragment mindestens 100 Aminosäuren umfasst.

4. Verwendung nach einem beliebigen der Ansprüche 1 bis 3, wobei die Pilzwirtszelle ausgewählt ist unter *Aspergillus sp., Trichoderma sp., Penicillium sp., Fusidium sp., Fusarium sp., Schizophyllum sp., Mucor sp.* und *Rhizopus sp.*

5. Verwendung nach Anspruch 4, wobei die *Aspergillus sp.* ausgewählt ist aus A. *nidulans, A. niger, A. awamori* und *A. oryzae.*

6. Verwendung nach Anspruch 4, wobei die *Trichoderma sp.* ausgewählt ist aus *T. reeseii, T. longibrachiatum* und *T. viride.*

7. Verwendung nach Anspruch 4, wobei die *Penicillium sp.* ausgewählt ist aus *P. notatum* und *P. chrysogenum.*

8. Verwendung nach einem beliebigen der Ansprüche 1 bis 3, wobei die Pilzwirtszelle ausgewählt ist unter: *Saccharomyces sp., Schizosaccharomyces sp., Zygosaccharomyces sp. Kluyveromyces sp., Candida sp., Pichia sp.* und *Hansenula sp.*

9. Verwendung nach Anspruch 8, wobei die *Saccharomyces sp.* ausgewählt ist aus *S. cerevisiae* und *S. ovarum.*

10. Verwendung nach Anspruch 8, wobei die *Schizosaccharomyces sp. S. pombe* ist.

11. Verwendung nach Anspruch 8, wobei die *Kluyveromyces sp. K. lactis* ist.

12. Verwendung nach Anspruch 8, wobei die *Candida sp. C. albicans* ist.

## Revendications

1. Utilisation d'une orientation $V_HV_L$ des domaines $V_L$ et $V_H$ d'une séquence scFv dans un polypeptide pour produire un polypeptide qui, quand il est exprimé dans une cellule hôte fongique, présente un taux inférieur de glycosylation par rapport à un polypeptide qui comprend les domaines $V_L$ et $V_H$ de la séquence scFv dans l'orientation $V_LV_H$.

2. Utilisation selon la revendication 1, dans laquelle le polypeptide est un scFv ou une fusion d'un scFV à une protéine plasmatique ou un fragment de celle-ci, ledit fragment comprenant au moins 100 acides aminés.

3. Utilisation selon la revendication 2, dans laquelle la protéine plasmatique est l'albumine ou un fragment de celle-ci, ledit fragment comprenant au moins 100 acides aminés.

4. Utilisation selon l'une quelconque des revendications 1 à 3, dans laquelle la cellule hôte fongique est choisie parmi : *Aspergillus sp., Trichoderma sp., Penicillum sp., Fusidium sp., Fusarium sp., Scizophyllum sp., Mucor sp.* et *Rhizopus sp.*.

5. Utilisation selon la revendication 4, dans laquelle *Aspergillus sp.* est choisi parmi *A. nidulans, A. niger, A. Awamori* et *A. oryzae.*

6. Utilisation selon la revendication 4, dans laquelle *Trichoderma sp.* est choisi parmi *T. reeseii, T. Longibrachiatum* et *T. viridee*.

7. Utilisation selon la revendication 4, dans laquelle *Penicillum sp.* est choisi parmi *P. notatum* et *P. chrysogenum*.

8. Utilisation selon l'une quelconque des revendications 1 à 3, dans laquelle la cellule hôte fongique est choisie parmi : *Saccharomyces sp., Schizosaccharomyces sp., Zygosaccharomyces sp., Klyveromyces sp., Candida sp., Pichia sp.* et *Hansenula sp.*.

9. Utilisation selon la revendication 8, dans laquelle *Saccharomyces sp.* est choisie parmi *S. cerevisiae* et *S. ovarum.*

10. Utilisation selon la revendication 8, dans laquelle *Schizosaccharomyces sp.* est *S. pombe.*

11. Utilisation selon la revendication 8, dans laquelle *Klyveromyces sp.* est *K. lactis.*

12. Utilisation selon la revendication 8, dans laquelle *Candida sp.* est *C. albicans.*

Figure 1

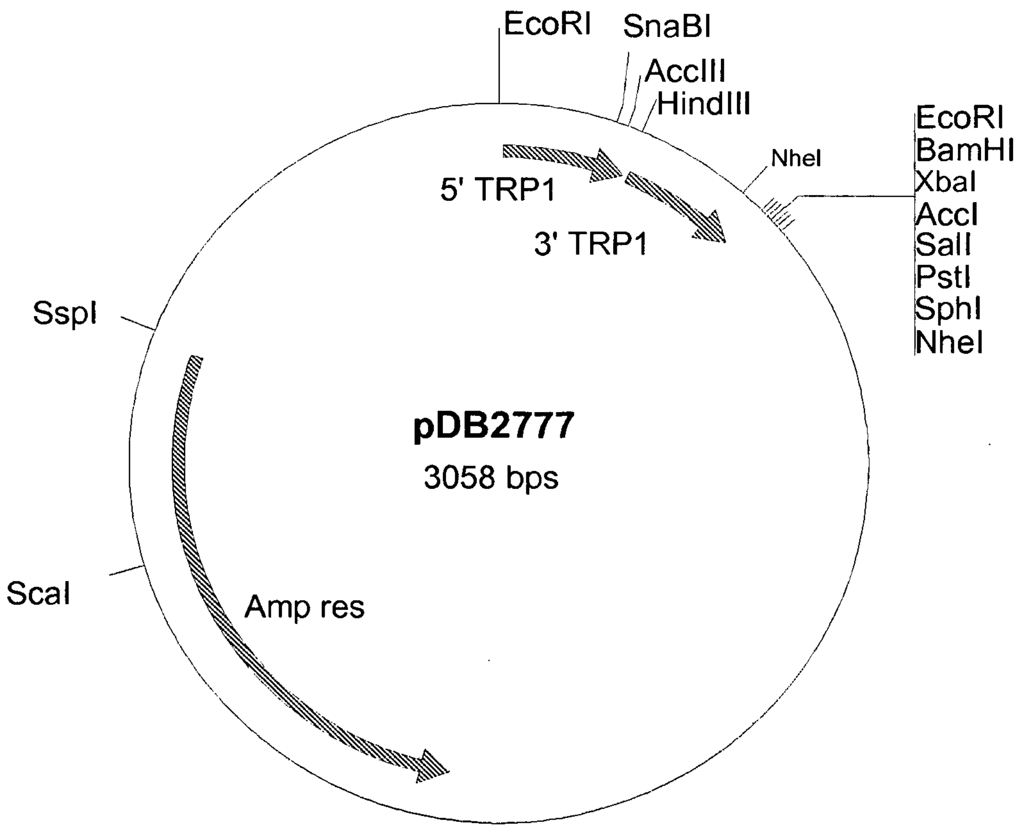
EcoRI
SnaBI
AccIII
HindIII
NheI
EcoRI
BamHI
XbaI
AccI
SalI
PstI
SphI
NheI
5' TRP1
3' TRP1
SspI
pDB2777
3058 bps
ScaI
Amp res

Figure 2

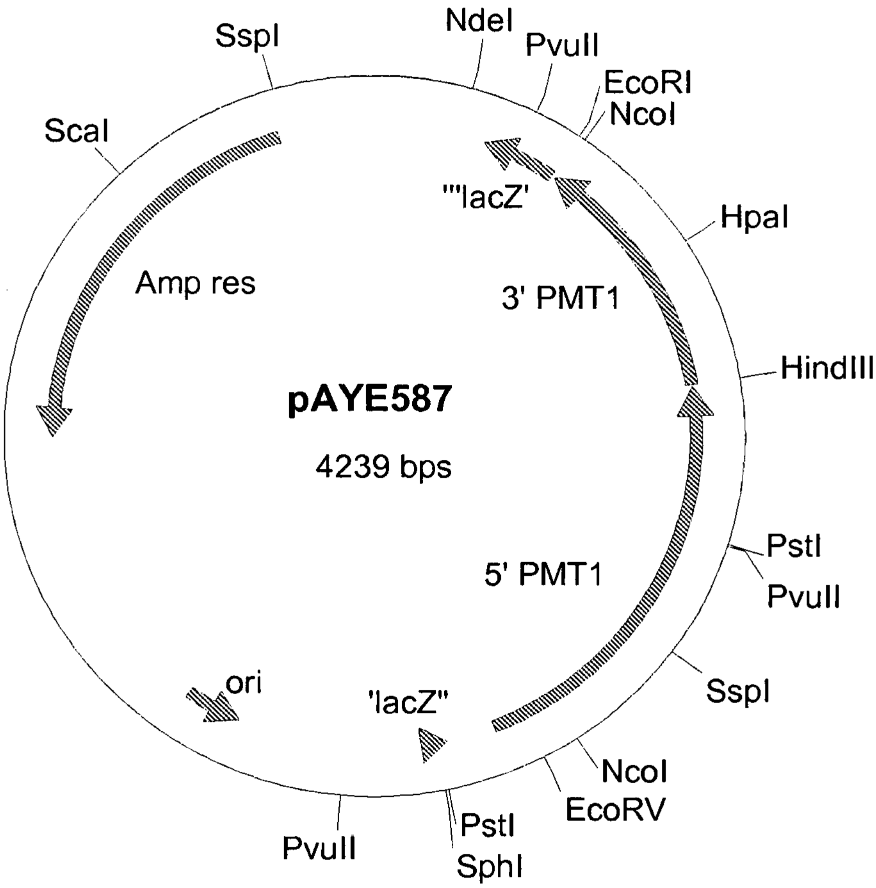
NdeI
SspI
PvuII
EcoRI
NcoI
ScaI
'''lacZ'
HpaI
Amp res
3' PMT1
HindIII
pAYE587
4239 bps
PstI
5' PMT1
PvuII
ori
SspI
'lacZ''
NcoI
EcoRV
PvuII
PstI
SphI

Figure 3

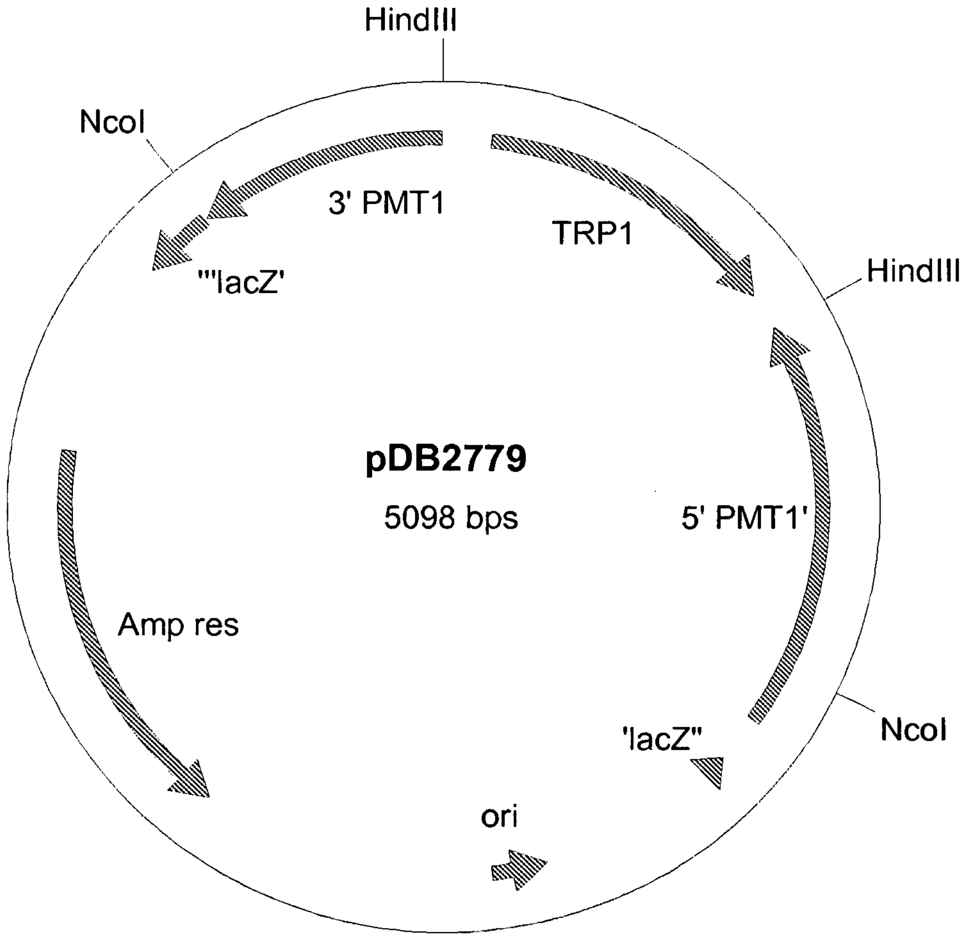
HindIII
NcoI
3' PMT1
TRP1
"'lacZ'
HindIII
pDB2779
5098 bps
5' PMT1'
Amp res
NcoI
'lacZ"
ori

Figure 4

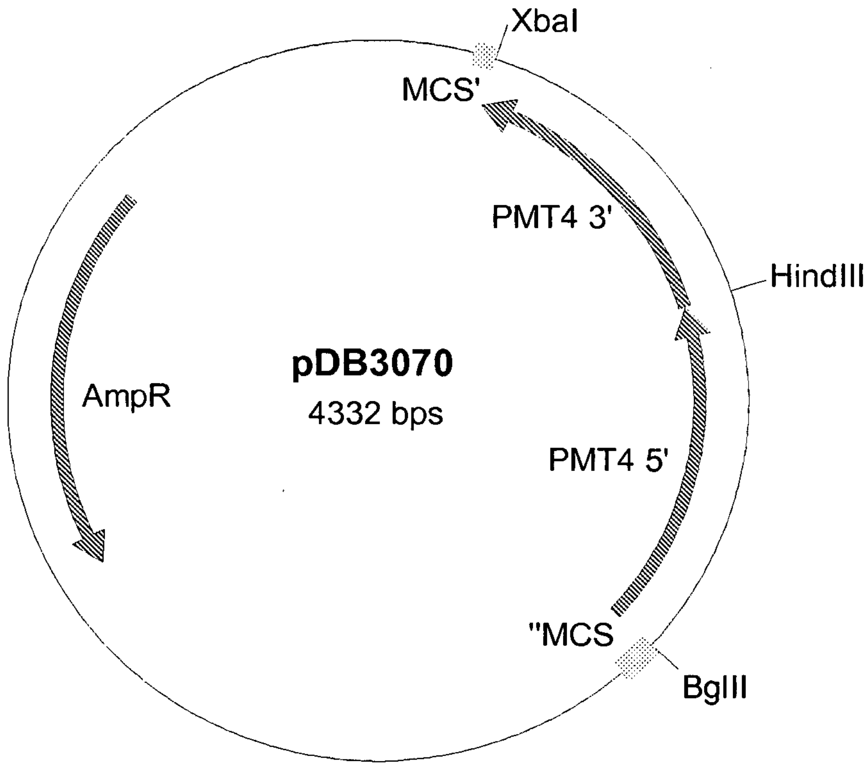
XbaI
MCS'
PMT4 3'
HindIII
pDB3070
4332 bps
AmpR
PMT4 5'
"MCS
BglII

Figure 5

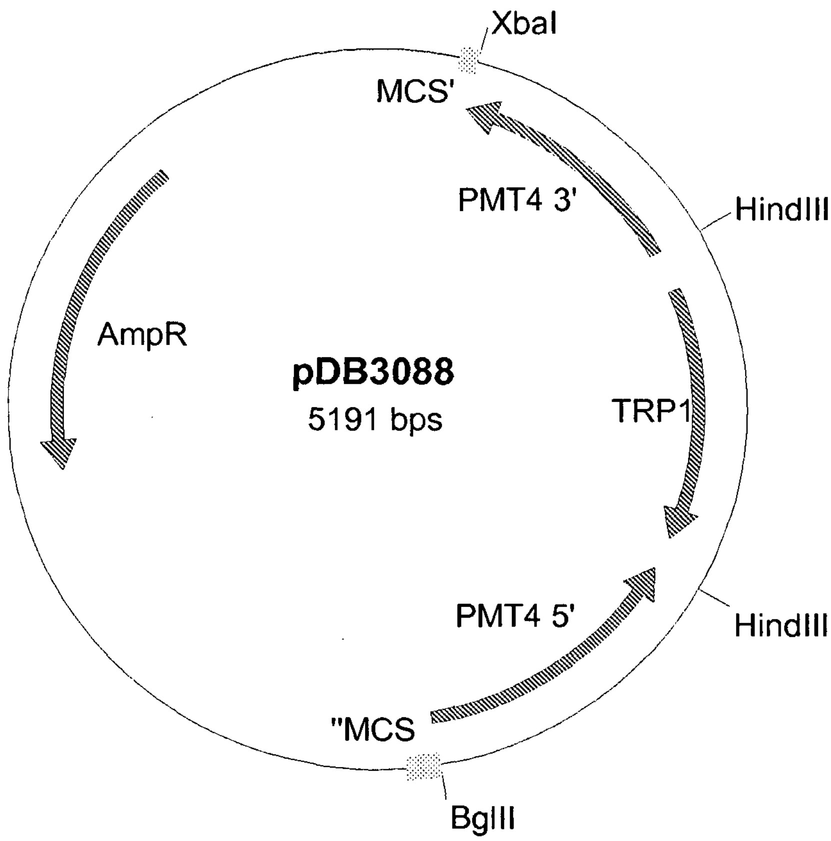
XbaI
MCS'
PMT4 3'
HindIII
AmpR
pDB3088
5191 bps
TRP1
PMT4 5'
HindIII
"MCS
BglII

Figure 6

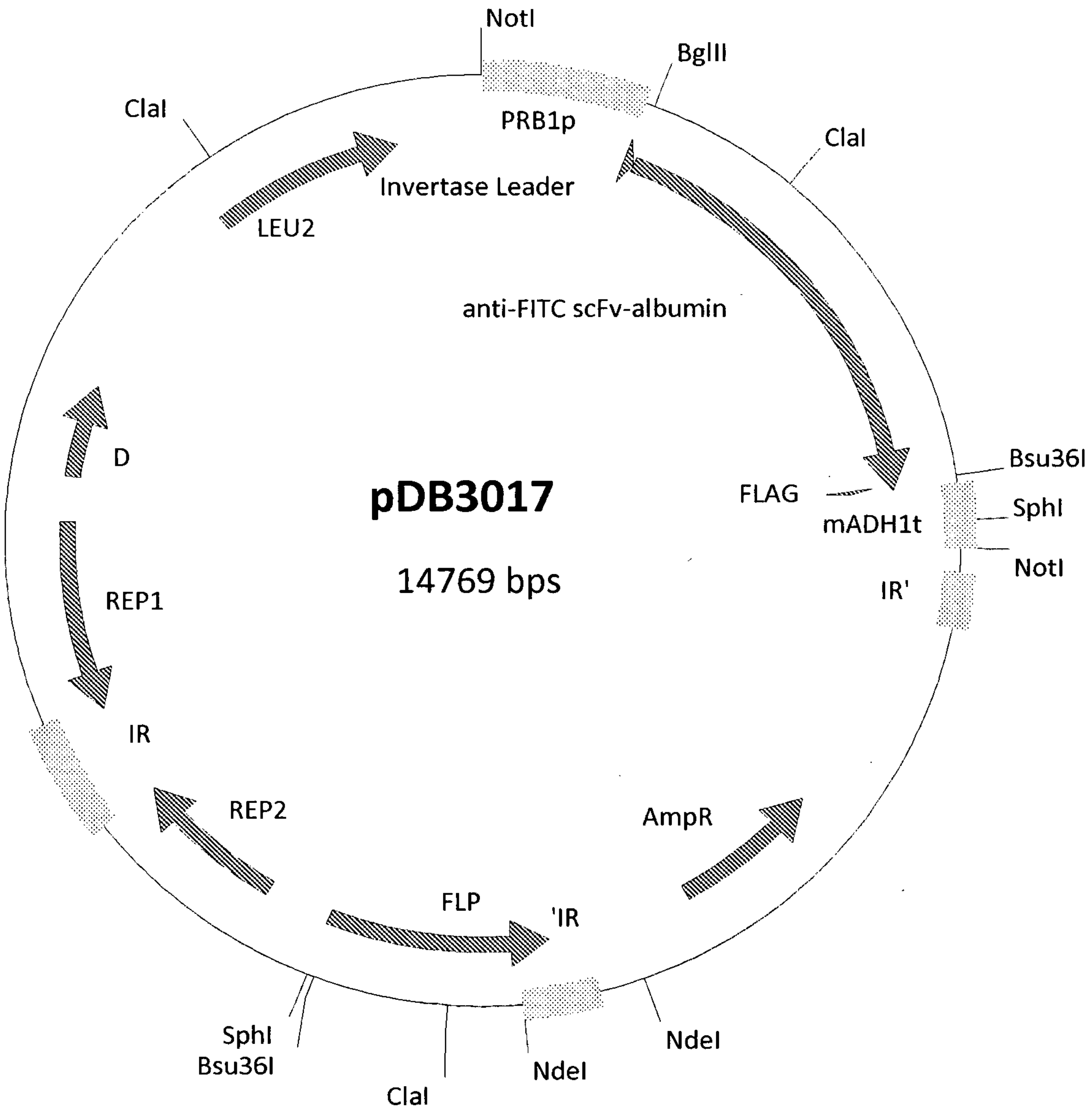
NotI
BglII
ClaI
PRB1p
ClaI
Invertase Leader
LEU2
anti-FITC scFv-albumin
D
pDB3017
14769 bps
FLAG
mADH1t
Bsu36I
SphI
NotI
IR'
REP1
IR
REP2
AmpR
FLP
'IR
SphI
Bsu36I
ClaI
NdeI
NdeI

Figure 7

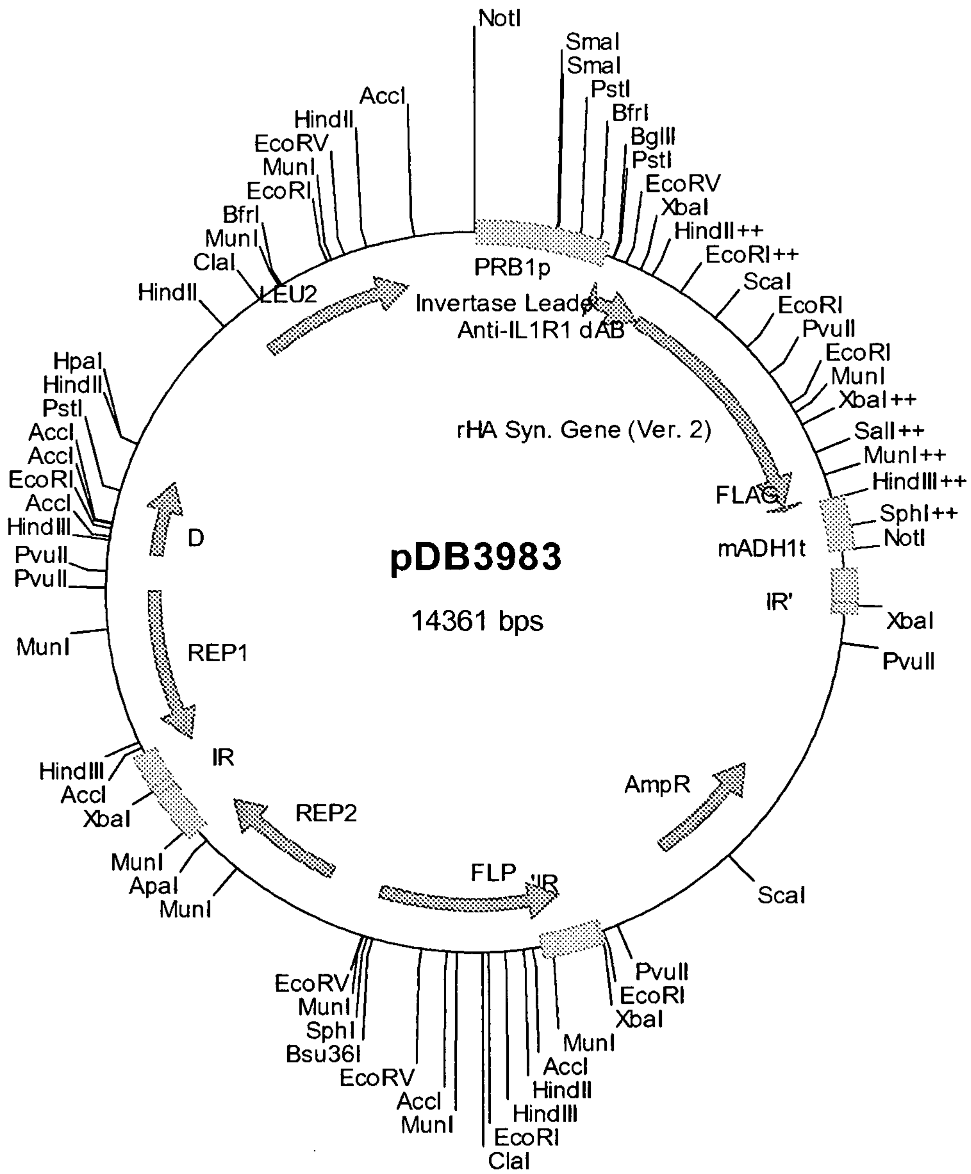
pDB3983
14361 bps
NotI
SmaI
SmaI
PstI
BfrI
BglII
PstI
EcoRV
XbaI
HindII++
EcoRI++
ScaI
EcoRI
PvuII
EcoRI
MunI
XbaI++
SalI++
MunI++
HindIII++
SphI++
NotI
XbaI
PvuII
ScaI
PvuII
EcoRI
XbaI
MunI
AccI
HindII
HindIII
EcoRI
ClaI
MunI
AccI
EcoRV
Bsu36I
SphI
MunI
EcoRV
MunI
ApaI
MunI
XbaI
AccI
HindIII
MunI
PvuII
PvuII
HindIII
AccI
EcoRI
AccI
AccI
PstI
HindII
HpaI
HindII
ClaI
MunI
BfrI
EcoRI
MunI
EcoRV
HindII
AccI
PRB1p
Invertase Leader
Anti-IL1R1 dAB
rHA Syn. Gene (Ver. 2)
FLAG
mADH1t
IR'
AmpR
FLP
IR
REP2
IR
REP1
D
LEU2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9429457 A
- WO 2007061631 A
- WO 2009105357 A
- WO 0029004 A
- WO 2009026638 A
- WO 2005118629 A
- US 20080220002 A
- WO 0179442 A
- WO 2009019314 A
- US 20060241027 A
- WO 0044772 A
- WO 2005061718 A


### Non-patent literature cited in the description


- **JOOSTEN et al.** *Microbial Cell Factories,* 2003, vol. 2, 1-15
- **GASSER ; MATTANOVICH.** *Biotechnol. Lett,* 2007, vol. 29, 201-212
- **MILLER et al.** *Protein Expression and Purification,* 2005, vol. 42, 255-267
- **LUSSIER, M. et al.** *Biochim. Biophys. Acta,* 1999, vol. 1426, 323-334
- **STRAHL-BOLSINGER, S. ; SCHEINOST, A.** *J. Biol. Chem.,* 1999, vol. 274, 9068-9075
- **WILLER et al.** *Curr. Opin. Struc. Biol.,* 2003, vol. 13, 621-630
- **LENGELER et al.** *Cell. Mol. Life Sci.,* 2008, vol. 65, 528-544
- **GIRRBACH, V. et al.** *J. Biol. Chem.,* 2000, vol. 275, 19288-19296
- **GIRRBACH, V ; STRAHL, S.** *J. Biol. Chem.,* 2003, vol. 278, 12554-12562
- **GOTO, M.** *Biosci. Biotechnol. Biochem.,* 2007, vol. 71, 1415-1427
- **TANAKA et al.** *Biochem. Biophys Res. Comm.,* 2005, vol. 330, 813-820
- **PRILL et al.** *Mol. Micro.,* 2005, vol. 55, 546-560
- **KURODA, K. et al.** *Appl. Environ. Microbiol.,* 2008, vol. 74, 446-453
- **FINCK et al.** *Glycobiology,* 1996, vol. 6, 313-320
- **GENTZSCH ; TANNER.** *Glycobiology,* 1997, vol. 7, 481-486
- **NEEDLEMAN ; WUNSCH.** *J. Mol. Biol.,* 1970, vol. 48, 443-453
- **RICE et al.** EMBOSS: The European Molecular Biology Open Software Suite. *Trends in Genetics,* 2000, vol. 16, 276-277, http://emboss.org
- **RICE et al.** *EMBOSS: The European Molecular Biology Open Software Suite,* 2000, http://emboss.org
- **ITO et al.** *J. Bacteriol.,* 1983, vol. 153, 16
- **ELBLE.** *Biotechniques,* 1992, vol. 13, 18
- **LENGELER K.B. et al.** *Cell Mol. Life Sci.,* 2008, vol. 65, 528-544
- **KERRY-WILLIAMS et al.** *Yeast,* 1998, vol. 14, 161-169
- **YANISCH-PERRON et al.** *Gene,* 1985, vol. 33, 103-119
- **TOYN et al.** *Yeast,* 2000, vol. 16, 553-560
- **BOLIVAR et al.** *Gene,* 1977, vol. 2, 95-113
- **HOHEISEL, J.** *BioTechniques,* 1994, vol. 17 (3), 456-459
- **LANTTO ; OHLIN.** *J. Biol. Chem.,* 2002, vol. 277, 45108-45114
- **S. M. KERRY-WILLIAMS et al.** *14,* 1998, vol. Yeast, 161-1690
- **ITO et al.** Sigma yeast transformation kit, YEAST-1, protocol 2. *J. Bacteriol.,* 1983, vol. 153, 16
- **SLEEP et al.** *Yeast,* 2002, vol. 18, 403
- Production of secreted proteins in yeast. **COLLINS, S.H.** Protein production by biotechnology. Elsevier, 1990, 61-77

SEQUENCE LISTING

<110> Novozymes Biopharma UK Ltd.

<120> Method of controlling glycosylation of antibodies

<130> 11585.204-WO

<160> 12

<170> PatentIn version 3.3

<210> 1
<211> 389
<212> DNA
<213> Artificial

<220>
<223> Synthetic

<400> 1

```
gaattcaatc agtaaaaatc aacggttaac gacattacta tatatataat ataggaagca      60
tttaatagaa cagcatcgta atatatgtgt actttgcagt tatgacgcca gatggcagta     120
gtggaagata ttctttattg aaaaatagct tgtcacctta cgtacaatct tgatccggag     180
cttttctttt tttgaagctt taaagataat gctaaatcat ttggcttttt gattgattgt     240
acaggaaaat atacatcgca ggggggttgac ttttaccatt tcaccgcaat ggaatcaaac    300
ttgttgaaga gaatgttcac aggcgcatac gctacaatga cccgattctt gctagccttt     360
tctcggtctt gcaaacaacc gccgaattc                                        389
```

<210> 2
<211> 1328
<212> DNA
<213> Artificial

<220>
<223> Synthetic

<400> 2

```
ccatggtcac tcttaaagag aagctgttag tggcctgtct tgctgtcttt acagcggtca      60
ttagattgca tggcttggca tggcctgaca gcgtggtgtt tgatgaagta catttcggtg     120
ggtttgcctc gcaatacatt agggggactt acttcatgga tgtgcatcct cctcttgcaa     180
agatgttgta tgctggtgtg gcatcgcttg gtgggttcca gggtgatttt gacttcgaaa     240
atattggtga cagctttcca tctacgacgc catacgtgtt gatgagattt ttctctgctt     300
ctttgggggc tcttactgtt attttgatgt acatgacttt acgttattct ggtgttcgta     360
tgtgggttgc tttgatgagc gctatctgct ttgccgttga aaactcgtac gtcactattt     420
ctcgttacat tctgttggac gccccattga tgtttttcat tgcagctgca gtctactctt     480
tcaagaaata cgaaatgtac cctgccaact cgctcaatgc ttacaagtcc ttgcttgcta     540
ctggtattgc tcttggtatg gcatcttcat ccaaatgggt tggtcttttc acggttacat     600
```

gggtgggtct tttatgtatc tggagactat ggttcatgat tggggatttg actaagtctt 660
ccaagtccat cttcaaagta gcatttgcca aattggcctt cttgttgggt gtgccttttg 720
ccctttatct ggtcttcttt tatatccact tccaatcatt aactttggac ggggatggcg 780
caagcttcat ttctaaattt attgaatccc ataaaaagat gtggcatatc aataaaaatt 840
tggtcgaacc tcatgtttat gaatcacaac caacttcatg gccattcttg ctacgtggta 900
taagttactg ggtgaaaat aacagaaacg tctatctatt aggtaatgcg atcgtatggt 960
gggctgtcac cgctttcatc ggtattttcg gattgattgt tatcactgag ctgttctcgt 1020
ggcagttagg taaaccaatt ttgaaggact ccaaggttgt taacttccac gttcaggtta 1080
ttcactactt attgggtttt gccgtccatt atgctccatc tttcttaatg caacgtcaaa 1140
tgtttttgca tcactactta cctgcttatt atttcggtat tcttgccctt ggccacgcct 1200
tggacataat agtttcttat gttttccgca gcaagagaca aatgggctac gcggtagtga 1260
tcactttcct tgctgcttct gtgtatttct tcaagagctt cagtccaatt atttacggta 1320
caccatgg 1328

<210> 3
<211> 865
<212> DNA
<213> Artificial

<220>
<223> Synthetic

<400> 3

```
aagctttcgg tcgaaaaaag aaaaggagag ggccaagagg gagggcattg gtgactattg    60
agcacgtgag tatacgtgat taagcacaca aaggcagctt ggagtatgtc tgttattaat   120
ttcacaggta gttctggtcc attggtgaaa gtttgcggct tgcagagcac agaggccgca   180
gaatgtgcac tagattccga tgctgacttg ctgggtatta tatgtgtgcc caatagaaag   240
agaacaattg acccggttat tgcaaggaaa atttcaagtc ttgtaaaagc atataaaaat   300
agttcaggca ctccgaaata cttggttggc gtgtttcgta atcaacctaa ggaggatgtt   360
ttggctctgg tcaatgatta cggcattgat atcgtccaac tgcatggaga tgagtcgtgg   420
caagaatacc aagagttcct cggtttgcca gttattaaaa gactcgtatt tccaaaagac   480
tgcaacatac tactcagtgc agcttcacag aaacctcatt cgtttattcc cttgtttgat   540
tcagaagcag gtgggacagg tgaacttttg gattggaact cgatttctga ctgggttgga   600
aggcaagaga gcccccgagag cttacatttt atgttagctg gtggactgac gccagaaaat   660
gttggtgatg cgcttagatt aaatggcgtt attggtgttg atgtaagcgg aggtgtggag   720
acaaatggtg taaaagactc taacaaaata gcaaatttcg tcaaaaatgc taagaaatag   780
gttattactg agtagtattt atttaagtat tgtttgtgca cttgcctgca agccttttga   840
aaagcaagca taaaagatca agctt                                          865
```

<210> 4
<211> 1378
<212> DNA
<213> Artificial

<220>
<223> Synthetic

<400> 4

```
agatctggta tccaaaagaa gttgttttg atgaggtaca tttcgggaaa tttgcatcgt      60
attacttaga aaggtcttat ttctttgacg ttcatccccc ttttgctaag atgatgattg    120
ccttcattgg ttggttatgt ggctatgatg gttcctttaa gtttgatgag attgggtatt    180
cttatgaaac tcatccagct ccatatatcg cgtaccgttc tttcaacgcg atattgggca    240
cattgactgt accaattatg ttcaacactt tgaaggaact gaatttcagg gctattacat    300
gtgcgtttgc atctctcttg gttgcaatcg atactgcgca tgttacagaa actaggctga    360
ttttactgga tgccatcttg attatttcta ttgctgctac tatgtattgt tacgttcgtt    420
tctacaagtg ccaattgcgt caacctttta catggagttg gtatatttgg ttacacgcta    480
ctggtttgtc tttatccttc gtgatttcca caaaatatgt tggtgttatg acatattccg    540
ctattggttt tgctgctgtg gtcaacttat ggcaattact ggacatcaag gcgggtttgt    600
ccttgaggca gttcatgaga cattttagta aaaggctgaa tggtttagtt ttgattccat    660
ttgtgattta cttgttttgg ttctgggttc atttcaccgt tttgaatact tcaggtcctg    720
gcgacgcaag cttaagccat taccattctt gaagaaatgg attgaaactc aaaaatctat    780
gttcgaacat aacaataaac tatcatcaga gcatccattt gcctctgaac cttacagttg    840
gcccggtagt ttaagtggtg tttcgttctg gaccaacggt gacgaaaaga agcaaatata    900
tttcattggt aacatcattg ggtggtggtt ccaagtcata tcattggctg tttttgttgg    960
cattatcgtg gccgatttaa ttactagaca tcgtggctat tatgccctaa acaagatgac   1020
cagagaaaag ctgtatggcc cattgatgtt tttcttcgtc tcctggtgct gtcattattt   1080
tccattcttt ttaatggcgc gtcaaaagtt tttgcatcat tacttaccag ctcatttaat   1140
cgcgtgctta ttctcaggag cactatggga agtaattttc agtgattgca aatcattgga   1200
tttggagaaa gacgaggata tttcaggtgc atcatatgaa cggaacccta aggtctacgt   1260
taaaccctat accgtcttct tggtgtgtgt ctcctgtgct gttgcgtggt tttttgtata   1320
cttttcacca ctagtgtatg gagatgtcag cttgtcacca tcggaagttg tttctaga     1378
```

<210> 5
<211> 54
<212> DNA
<213> Artificial

<220>
<223> Primer LES 21

<400> 5
gttggtcgct gcttcccaag ctgccttagg tttgtaataa gcttaattct tatg 54

<210> 6
<211> 54
<212> DNA

<213> Artificial

<220>
<223> Primer LES 22

<400> 6
cataagaatt aagcttatta caaacctaag gcagcttggg aagcagcgac caac 54

<210> 7
<211> 38
<212> DNA
<213> Artificial

<220>
<223> Primer Fwd rHA single FLAG

<400> 7
ttaggcttag attataaaga tgatgacgat aaataata 38

<210> 8
<211> 39
<212> DNA
<213> Artificial

<220>
<223> Primer Rv rHA single FLAG

<400> 8
agcttattat ttatcgtcat catctttata atctaagcc 39

<210> 9
<211> 774
<212> DNA
<213> Artificial

<220>
<223> Synthetic

<400> 9

**agatctctgc agaagttcaa ttgttggaat ctggtggtgg tttggttcaa cctggtggtt 60**

**ctttgagatt gtcttgtgct gcttctggtt ttactttttc taattattgg atgtcttggg 120**

**ttagacaagc tccaggtaaa ggtttggaat gggtttccgg tatttcaggt aatggtggtt 180**

**atacttattt tgctgattca gttaaagata gatttactat ttctagagat aattctaaaa 240**

**ataccttata tttgcaaatg aactctttga gagcagaaga tactgctgtt tattactgtg 300**

**caggtggtga cggttctggt tggagttttt ggggtcaagg tactctagtt accgtttctt 360**

**caggtggtgg tggttctggt ggaggtggat caggtggtgg aggatctcaa tcagttttga 420**

**ctcaaccacc atctgcttca ggtactccag gtcaaagagt taccatttct tgtactggtt 480**

cttcttctaa tattggtgca ggttacgatg ttcattggta tcaacaattg ccaggtactg 540

ctccaaaatt gttgatttat ggtaacaaca atagaccatc tggtgtccca gatagatttt 600

ctggttctaa atctggtact tctgcttctt tggctatttc tggtttaaga tcagaagatg 660

aagctgatta ctactgtgct gcttgggatg actctttgtc tggtagagtt ttcggtggtg 720

gtactaaatt gaccgttttg ggtgacgctc acaagtccga agtcgctcat cgat 774

<210> 10
<211> 91
<212> DNA
<213> Artificial

<220>
<223> Primer CF 138

<400> 10

agcttaacct aattctaaca agcaaagatg cttttgcaag ccttcctttt ccttttggct 60

ggttttgcag ccaagatctc tgcagaagac a 91

<210> 11
<211> 91
<212> DNA
<213> Artificial

<220>
<223> Primer CF 139

<400> 11

agcttgtctt ctgcagagat cttggctgca aaaccagcca aaaggaaaag gaaggcttgc 60

aaaagcatct ttgcttgtta gaattaggtt a 91

<210> 12
<211> 108
<212> PRT
<213> Artificial

<220>
<223> synthetic

<400> 12

```
Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Ile Tyr Leu Asn
            20                  25                  30

Leu Asp Trp Tyr Gln Gln Lys Pro Gly Lys Ala Pro Lys Leu Leu Ile
        35                  40                  45

Tyr Gly Thr Ser Asp Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Pro Ser Phe Tyr Phe Pro Tyr
                85                  90                  95

Thr Phe Gly Gln Gly Thr Lys Val Glu Ile Arg Arg
            100                 105
```